(19) Europäisches Patentamt
European Patent Office
Office européen des brevets

(11) EP 3 254 653 B1

(12) **EUROPEAN PATENT SPECIFICATION**

(45) Date of publication and mention
of the grant of the patent:
**29.05.2019 Bulletin 2019/22**

(51) Int Cl.:
***A61F 13/49*** *(2006.01)*     ***A61F 13/511*** *(2006.01)*
***A61F 13/512*** *(2006.01)*

(21) Application number: **15894279.7**

(22) Date of filing: **26.08.2015**

(86) International application number:
**PCT/JP2015/074093**

(87) International publication number:
**WO 2016/194247 (08.12.2016 Gazette 2016/49)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.05.2015 JP 2015110913**

(43) Date of publication of application:
**13.12.2017 Bulletin 2017/50**

(73) Proprietor: **Unicharm Corporation
Ehime 799-0111 (JP)**

(72) Inventors:
• **UDA, Masashi
Kanonji-shi
Kagawa 769-1602 (JP)**
• **SAKAGUCHI, Satoru
Kanonji-shi
Kagawa 769-1602 (JP)**
• **MIYAMA, Takuya
Kanonji-shi
Kagawa 769-1602 (JP)**

(74) Representative: **Dolleymores
9 Rickmansworth Road
Watford, Hertfordshire WD18 0JU (GB)**

(56) References cited:
EP-A1- 2 277 485     EP-A1- 2 505 173
EP-A1- 2 612 961     WO-A1-2013/005782
JP-A- 2003 102 772   JP-A- 2006 129 891
JP-A- 2006 217 951   JP-A- 2006 320 355
JP-A- 2006 320 355   JP-A- 2009 273 722
JP-A- 2011 131 044   JP-B2- 3 986 505

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to an absorbent article, such as a sanitary napkin, disposable diaper or incontinence pad.

Background Art

Among absorbent articles, such as disposable diapers and sanitary napkins, absorbent articles have been investigated that employ cotton nonwoven fabrics including cotton as natural fibers, for the structural members, such as top sheet, in order to obtain the feeling of assurance that is provided by natural materials.

As an example of such an absorbent article, PTL 1 proposes an absorbent article wherein, there being provided a top sheet constructed of a cotton nonwoven fabric and an absorbent body as a lower layer below the top sheet, there is inserted between them a heat-fusible fiber sheet having lower fiber density and hydrophilicity than the cotton nonwoven fabric, and in this layered state, numerous embossings are formed from the top sheet surface side. The absorbent article disclosed in PTL 1 is thought to allow moisture absorbed into the top sheet to rapidly penetrate into the interior, without impairing flexibility.

Citation List

Patent Literature

[PTL 1] Japanese Unexamined Patent Publication No. 2009-148328
EP 2277485 A1 discloses a top sheet for an absorbent article having a plurality of fused compressed sections. The top sheet may be composed of two layers.

JP 2006 320355 A relates to a surface sheet for an absorbent article, the sheet having an upper fiber layer located on the front surface side, a lower fiber layer located on the rear surface side, and projections and recesses.

WO 2013/005782 A1 discloses an absorbent article having a top sheet, a back sheet, an absorbent core and a cover sheet covering the absorbent core. The non-skin contact surface side of the top sheet has a plurality of back recesses. The cover sheet enters the back recesses to provide a space between the cover sheet and the absorbent core.

EP 2505173 A1 relates to an absorbent article comprising a liquid permeable top sheet, a liquid impermeable or water repellent back sheet, and an absorbent member arranged between the top sheet and the back sheet, the top sheet being formed of a nonwoven sheet having high density portions and low density portions, the high density portions and the low density portions being arranged in planar directions of the top sheet, and the low density portions having a larger amount of a skin care agent applied thereto than the high density portions.

Summary of Invention

Technical Problem

However, when a cotton nonwoven fabric with high water retention is provided as a top sheet on the upper side of an absorbent body, as in the absorbent article disclosed in PTL 1, and excreted fluid such as urine absorbed into the absorbent body has been discharged from the absorbent body as moisture by evaporation and the like, it becomes absorbed and retained in the cotton nonwoven fabric provided on the upper side of the absorbent body, such that the wearer perceives a condition of dampness or feels mustiness, potentially producing a feeling of discomfort.

It is therefore an object of the present invention to provide an absorbent article using a nonwoven fabric including cotton as the top sheet, wherein issues such as causing perception of a condition of dampness by the wearer, a musty feel, or discomfort for the wearer are unlikely to occur.

Solution to Problem

The absorbent article according to one aspect of the invention (aspect 1) is an absorbent article including a liquid-permeable top sheet situated on the side facing the skin of the wearer, a liquid-impermeable back sheet situated on the side not facing the skin of the wearer, and an absorbent body situated between these sheets, wherein the top sheet is a nonwoven fabric comprising at least two fiber layers including a first fiber layer composed of cotton and thermoplastic resin fibers and a second fiber layer composed of hydrophobic thermoplastic resin fibers, the nonwoven fabric having a first surface which is the skin-facing side surface, formed by the second fiber layer, and a second surface which is the non-skin-facing side surface, facing the absorbent body, the nonwoven fabric being provided with a plurality of protrusions protruding toward the first surface side and a plurality of recesses depressed toward the second surface side, and formed between adjacent protrusions, each of the protrusions having an empty space faced by the second surface of the nonwoven fabric.

According to the absorbent article of aspect 1, the top sheet is a nonwoven fabric comprising at least two fiber layers, including a first fiber layer composed of cotton and thermoplastic resin fibers, and a second fiber layer composed of hydrophobic thermoplastic resin fibers, the nonwoven fabric being provided with a plurality of protrusions protruding toward the first surface side and a plurality of recesses depressed toward the second surface side, and the each of the protrusions having empty space faced by the second surface of the nonwoven fabric, and therefore even when excreted fluid such as urine from a wearer absorbed into the absorbent body has been discharged from the absorbent body as moisture by evaporation and the like, the moisture is absorbed and held in the cotton of the first fiber layer while being retained in a state of moisture in the empty spaces (i.e. the empty spaces are in a highly humid state), such that a state of gas-liquid equilibrium is formed between the moisture in the empty spaces (the gas phase) and the excreted fluid absorbed and held in the absorbent body (the liquid phase), and further discharge of moisture from the absorbent body can be inhibited.

Furthermore, since the first surface of the nonwoven fabric, which is the surface facing the skin of the wearer, is formed by the second fiber layer, excreted fluid such as urine that has been absorbed and held in the cotton of the first fiber layer is prevented from contact with the surface of the skin of the wearer.

With the absorbent article of aspect 1, therefore, there is low likelihood of creating a perception of a condition of dampness or causing a musty feel or the like for the wearer, which could produce discomfort for the wearer.

An absorbent article according to another aspect of the invention (aspect 2) is the absorbent article of aspect 1 in which the back sheet has air permeability.

Since the absorbent article of aspect 2 has air permeability for the liquid-impermeable back sheet, moisture discharged from the absorbent body to the non-skin-facing side can be discharged through the back sheet, and it is possible to reduce moisture in the absorbent article or moisture trapped between the absorbent article and the surface of the skin of the wearer.

Thus, the absorbent article of aspect 2 can further help avoid a feeling of mustiness or the like for the wearer.

An absorbent article according to yet another aspect of the invention (aspect 3) is the absorbent article according to aspect 1 or 2 including a cotton fiber mass in the first fiber layer.

With the absorbent article of aspect 3, since the first fiber layer situated on the second surface (non-skin-facing surface) side of the nonwoven fabric includes a cotton fiber mass, moisture (excreted fluid) discharged from the absorbent body is absorbed and held in the cotton fiber mass in the first fiber layer in a concentrated (spot-like) manner, and it is possible to reduce the area of the sections where moisture has been absorbed and held in the in-plane direction of the first fiber layer (to a spot-like form), thereby making it possible to minimize the amount of moisture discharged from the first surface side of the first fiber layer. As a result, moisture discharged from the absorbent body can be effectively confined in the empty spaces of the protrusions.

An absorbent article according to yet another aspect of the invention (aspect 4) is the absorbent article according to any one of aspects 1 to 3 wherein the absorbent article has a back surface film on the non-skin-facing side of the back sheet, the back surface film having lower air permeability than the back sheet.

With the absorbent article of aspect 4, which has a back surface film with lower air permeability than the back sheet on the non-skin-facing side of the back sheet, an air permeability gradient is formed in which the air permeability is lower from the back sheet toward the back surface film, and moisture discharged from the absorbent body toward the non-skin-facing side (that is, the back sheet side) tends to be drawn into the back surface film through the back sheet, and be eliminated to the exterior of the absorbent article. As a result, it is possible to reduce moisture discharged from the absorbent body to the skin-facing side (i.e. the side opposite the back sheet side), and to reduce the likelihood of a feeling of mustiness or the like for the wearer.

An absorbent article according to yet another aspect of the invention (aspect 5) is the absorbent article according to any one of aspects 1 to 4, wherein the protrusions are laid out in a first direction on the first surface of the nonwoven fabric and provided at predetermined intervals in a second direction that is perpendicular to the first direction, the recesses are laid out in the first direction and provided between adjacent protrusions in the second direction, the recesses each having a first recess comprising a first bottom section situated more toward the second surface side than the location of the first surface side at the top sections of the protrusions, and a plurality of second recesses inside the first recess, provided in a discontinuous manner in the first direction, and depressed from the first bottom section toward the second surface, wherein the second recesses comprise perimeter wall sections extending from the first bottom section in the direction toward the second surface side, and second bottom sections having the highest fiber density of the nonwoven fabric, provided on the edges of the second surface sides of the perimeter wall sections, so as to plug the edges.

The absorbent article of aspect 5 is constructed so that the fiber density on the second bottom sections of the second recesses located between adjacent protrusions, in the nonwoven fabric composing the aforementioned top sheet, is the highest in the nonwoven fabric, and in the thickness direction of the nonwoven fabric, it has a density gradient from the protrusions with relatively low fiber density to the second bottom sections of the second recesses having the highest fiber density, such that excreted fluid such as urine supplied from the first surface side of the nonwoven fabric is easily drawn in a spot-like manner from the protrusions to the second bottom sections of the second recesses, allowing an excellent absorption property (especially absorption rate and liquid migration properties) to be exhibited as a top sheet, and helping to prevent moisture that has been discharged from the absorbent body, from migrating from the second surface side to the first surface side of the nonwoven fabric. As a result, moisture that has been discharged from the absorbent body can be effectively confined in the empty spaces of the protrusions, and the wearer can be even further protected from feeling mustiness and the like.

When the amount of cotton in the fiber layer is increased, the flexibility of the fiber layer is reduced by the rigidity of the cotton and the cushioning property and feel on the skin of the layered nonwoven fabric including the fiber layer, as well as its follow property for the shape of the body, may potentially be impaired, but with the absorbent article of aspect 5, due to the relatively low fiber density of the protrusions and the first recesses that tend to contact with the skin of the wearer, the fact that the sections that most easily tend to contact with the skin of the wearer are the top sections of the protrusions that have small contact area, and the fact that stress in the thickness direction acting from the first surface side of the nonwoven fabric can be buffered by deformation of the first recesses in the second surface side, it is possible to ensure adequate flexibility even when the top sheet is composed of a nonwoven fabric that includes cotton.

An absorbent article according to yet another aspect of the invention (aspect 6) is the absorbent article of aspect 5 wherein the pitch of the second recesses in the second direction is no greater than 2.0 mm.

With the absorbent article of aspect 6, since the pitch of the second recesses in the second direction is no greater than 2.0 mm, excreted fluid such as urine supplied from the first surface side of the nonwoven fabric is easily drawn up in a spot-like manner into the second bottom sections of the second recesses disposed between adjacent protrusions, such that the effect exhibited by the absorbent article of aspect 5 is exhibited even more prominently. In addition, in the absorbent article of aspect 6, the cotton in the first fiber layer is easily held in the second bottom sections of the second recesses, thereby helping to prevent tearing of the fiber layer at the second bottom sections and allowing the nonwoven fabric (top sheet) to have excellent strength.

An absorbent article according to yet another aspect of the invention (aspect 7) is the absorbent article according to any one of aspects 1 to 6 wherein the protrusions are laid out in the first direction of the first surface of the nonwoven fabric and are provided at predetermined intervals in a second direction that is perpendicular to the first direction, while the protrusions are laid out up to at least one of the edges in the first direction of the absorbent body, or exceeding at least one of the edges.

With the absorbent article of aspect 7, not only moisture discharged from the skin-facing side of the absorbent body, but also moisture discharged from the edges (side surfaces) in the first direction of the absorbent body can be confined to the empty spaces of the protrusions, and thus further help to protect the wearer from a feeling of mustiness or the like.

An absorbent article according to yet another aspect of the invention (aspect 8) is the absorbent article according to any one of aspects 1 to 7 wherein the absorbent body has compressed sections on the non-skin-facing side surface.

Since the absorbent article of aspect 8 has compressed sections on the non-skin-facing side surface of the absorbent body and excreted fluid absorbed into the absorbent body easily pools in the compressed sections, with little thereof remaining on the skin-facing side surface of the absorbent body, it is possible to reduce moisture discharged from the absorbent body to the skin-facing side, and to make it even less likely that the wearer will feel mustiness or the like.

An absorbent article according to yet another aspect of the invention (aspect 9) is the absorbent article according to aspect 8 wherein the absorbent article has a lengthwise direction, a widthwise direction and a thickness direction that are mutually perpendicular, the compressed sections are provided on the non-skin-facing side surface of the absorbent body, as linear compressed sections running in the lengthwise direction and/or the widthwise direction, and the absorbent body has a plurality of mutually spaced punctiform compressed sections on the skin-facing side surface.

Since the absorbent article of aspect 9 has linear compressed sections on the non-skin-facing side surface of the absorbent body, running in the lengthwise direction and/or the widthwise direction, and excreted fluid absorbed into the absorbent body easily pools in a state diffused along the linear compressed sections running in the lengthwise direction and/or the widthwise direction, with little thereof remaining on the skin-facing side surface of the absorbent body, it is possible to reduce moisture discharged from the absorbent body to the skin-facing side, and to make it even less likely that the wearer will feel mustiness.

Furthermore, since it has a plurality of punctiform compressed sections on the skin-facing side surface of the absorbent body, i.e. it has punctiform compressed sections that have high fiber density and function as liquid-absorbing sections utilizing capillary movement, excreted fluid such as urine is easily absorbed in a spot-like manner into the absorbent body and less discharged through the punctiform compressed sections to the skin-facing side, thereby allowing moisture discharged from the absorbent body to the skin-facing side to be reduced, and helping to further avoid a feeling of mustiness for the wearer.

An absorbent article according to yet another aspect of the invention (aspect 10) is the absorbent article according to any one of aspects 1 to 9 wherein the absorbent article has a lengthwise direction, a widthwise direction and a thickness direction that are mutually perpendicular, the top sheet having a center region running in the lengthwise direction and including the lengthwise axial line of the absorbent article, in a plane view, and a pair of outside regions located at both edges in the lengthwise direction of the center region and running in the lengthwise direction, the absorbent article having joining sections at the sections of the second surface sides of the recesses of the nonwoven fabric, that join with the absorbent body, the joining sections extending in the lengthwise direction and being disposed in a plurality of rows in the widthwise direction between the nonwoven fabric and the absorbent body, the joining sections being disposed so that, among the intervals between adjacent joining sections in the widthwise direction, the intervals between joining sections lying in the outside regions of the top sheet are larger than the intervals between joining sections lying in the center region in the widthwise direction of the top sheet.

With the absorbent article of aspect 10, the pitch of the joining sections lying in the center region of the top sheet to which excreted fluid such as urine has been supplied are disposed more densely than in the outside regions, and it is therefore possible to firmly bond the top sheet and the absorbent body at the center region that greatly contributes to absorption of excreted fluids, while also allowing the empty spaces of the protrusions at the center region to be formed denser and in greater number than in the outside regions, thereby helping to reliably ensure empty spaces that hold moisture discharged from the absorbent body.

Advantageous Effects of Invention

According to the invention, it is possible to provide an absorbent article using a nonwoven fabric including cotton as the top sheet, wherein issues such as causing perception of a condition of dampness by the wearer, musty feel or discomfort for the wearer are unlikely to occur.

Brief Description of the Drawings

Fig. 1 is a plan view of a disposable diaper according to an embodiment of the invention.
Fig. 2 is a magnified cross-sectional view of the disposable diaper according to the embodiment of the invention shown in Fig. 1, along line II-II'.
Fig. 3 is a perspective view of a nonwoven fabric (fiber laminate) composing the top sheet of a disposable diaper according to an embodiment of the invention.
Fig. 4 is a partial enlarged plan view of a top sheet of a disposable diaper according to the embodiment of the invention.
Fig. 5 is a magnified cross-sectional view of the disposable diaper according to the embodiment of the invention shown in Fig. 4, along line V-V'.
Fig. 6 is a magnified cross-sectional view of the disposable diaper according to the embodiment of the invention shown in Fig. 4, along line VI-VI'.
Fig. 7 is a magnified cross-sectional view of the disposable diaper according to the embodiment of the invention shown in Fig. 4, along line VII-VII'.
Fig. 8 is a schematic diagram for illustration of an example of a production apparatus for an absorbent article of the invention.
Fig. 9 is a partial magnified perspective view schematically showing a pair of shaping rolls of a shaping apparatus in a production apparatus for an absorbent article of the invention.
Fig. 10 is a partial magnified schematic diagram showing the arrangement of pins on the lower shaping roll of the pair of shaping rolls shown in Fig. 9.
Fig. 11 is a partial magnified schematic diagram showing the interlocked state of the upper shaping roll and lower shaping roll of the pair of shaping rolls shown in Fig. 9.

Description of Embodiments

Preferred embodiments of the absorbent article of the invention will now be described in detail with reference to the accompanying drawings. Throughout the present description, unless otherwise specified, the simple phrase "in a plane view" will be used to mean that the absorbent article in the expanded flat state is viewed from the upper side in the thickness direction. Also throughout the present description, the term "skin-facing side" means the side that is relatively near the skin surface of the wearer in the thickness direction of the absorbent article, when the wearer of the absorbent article has worn the absorbent article, and "non-skin-facing side" means the side that is relatively far from the skin surface of the wearer in the thickness direction of the absorbent article, when the wearer of the absorbent article has worn the absorbent article.

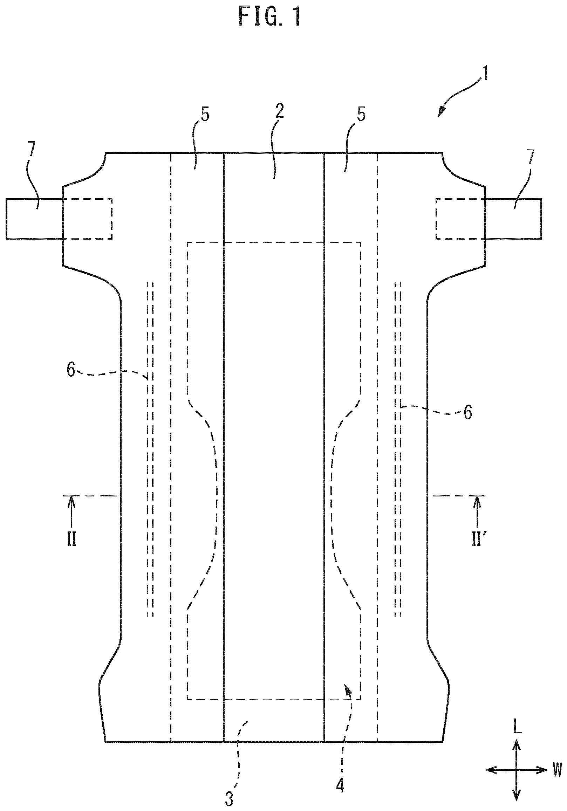

Fig. 1, Fig. 2 and Fig. 4 to Fig. 7 are diagrams schematically showing a disposable diaper 1 (absorbent article) according to an embodiment of the invention. The disposable diaper 1 has a lengthwise direction L, a widthwise direction W and a thickness direction T that are mutually perpendicular, and comprises a liquid-permeable top sheet 2 situated on the side facing the skin of the wearer, a liquid-impermeable back sheet 3 situated on the side not facing the skin of the wearer, and an absorbent body 4 situated between the two sheets.

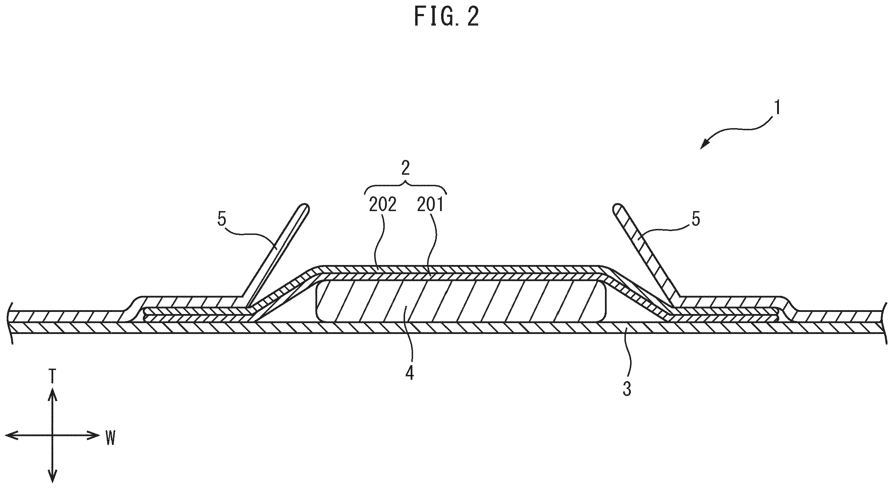

As shown in Fig. 1 and Fig. 2, the disposable diaper 1 comprises a pair of anti-leakage wall sections 5, 5 made of a pair of side section sheets, an elastic member 6 made of rubber or the like for stretching of the section around the left and right legs in contact with the femoral region of the wearer, in the lengthwise direction L, and connecting tape 7 for connection between the abdominal region and dorsal region of the disposable diaper when it is worn, there being further provided an outer sheet (not shown) on the non-skin-facing side of the back sheet 3.

The back sheet 3 is provided on the disposable diaper 1 on the side not facing the skin of the wearer (the lower side of the absorbent body 4 in Fig. 2), serving to prevent permeation of excreted fluid that has been discharged and to prevent its leakage into underwear or clothing. The back sheet 3 is not permeable to fluids, such as excreted fluids, but has a prescribed degree of air permeability. If the back sheet has air permeability, moisture discharged from the absorbent body to the non-skin-facing side can be discharged through the back sheet, and it is possible to reduce moisture in the absorbent article or moisture trapped between the absorbent article and the side of the skin of the wearer.

In addition, the back sheet 3 of this embodiment, while sandwiching the absorbent body 4 against the top sheet 2, is mutually bonded with the top sheet 2 at the perimeter sections.

The absorbent body 4 serves to absorb excreted fluid, such as urine, being one that contains an absorbent material that absorbs and holds excreted fluid. Moreover, the absorbent body 4 is formed long in the direction along the lengthwise direction L of the disposable diaper 1, both end sides in the lengthwise direction L being curved so as to be outwardly convex in the lengthwise direction of the absorbent body 4, and it is formed into an essentially oblong shape in a plane view, that has a constant thickness and is smaller than the top sheet 2 or back sheet 3. In the absorbent body 4 of this embodiment, both the surface of the top sheet side (that is, the side facing the skin of the wearer) and the surface of the back sheet side (that is, the side not facing the skin of the wearer) are formed flat.

In the absorbent article of the invention, the thickness, basis weight and other properties of the absorbent body are not particularly restricted, and may be adjusted as appropriate for the properties to be provided for an absorbent article, such as a disposable diaper (including absorption properties, strength and lightweight properties). For example, the thickness of the absorbent body will usually be in the range of 0.1 to 15 mm, and is preferably in the range of 1 to 10 mm and more preferably in the range of 2 to 5 mm, while the basis weight will usually be in the range of 20 to 1000 $g/m^2$, and is preferably in the range of 50 to 800 $g/m^2$ and more preferably in the range of 100 to 500 $g/m^2$. The thickness and basis weight of the absorbent body may be constant across the entire absorbent body, or it may partially differ.

Furthermore, for this embodiment, the absorbent body 4 has the top sheet 2 on the surface of the top sheet side (the upper side of the absorbent body 4 in Fig. 2) and the back sheet 3 on the surface of the back sheet side (the lower side of the absorbent body 4 in Fig. 2) bonded by an adhesive, such as a hot-melt adhesive.

The top sheet 2 contacts the skin of the wearer and causes rapid absorption or penetration of excreted fluids from the wearer, causing them to migrate toward the absorbent body 4, and it is disposed on the skin-facing side of the absorbent body 4, that faces the skin of the wearer (the upper side of the absorbent body 4 in Fig. 1, Fig. 2 and Fig. 4 to Fig. 7). The top sheet 2 is formed long in the direction along the lengthwise direction L of the disposable diaper 1.

For this embodiment, from the viewpoint of improving the feeling of assurance provided by natural materials and absorption properties, there is used as the top sheet 2 a nonwoven fabric comprising a two-layer fiber laminate 200, including a first fiber layer 201 composed of cotton and thermoplastic resin fibers, and a second fiber layer 202 composed of hydrophobic thermoplastic resin fibers, as a fiber layer adjacent to the surface of the upper side of the first fiber layer 201 and forming a first surface 2a (upper side) of the top sheet 2.

According to the invention, the nonwoven fabric composing the top sheet is not limited to a two-layer fiber laminate, and may be a fiber laminate comprising three or more fiber layers including the first fiber layer and the second fiber layer.

According to the invention, the cotton in the first fiber layer is not particularly restricted, and for example, cotton having a fineness in the range of 1.0 to 15 dtex and a fiber length in the range of 5 to 40 mm may be used. Cotton having a fiber length of 20 mm or greater is preferably used since, when the first fiber layer is laminated with the second fiber layer described below (that is, when the fiber laminate is formed), it will be possible for a portion of the cotton fibers to readily infiltrate into the second fiber layer, and to increase the liquid permeability of the nonwoven fabric. Also, blended cotton which is a blend of cotton having a fiber length of 20 mm or greater and cotton having a fiber length of 10 mm or smaller is particularly preferred for use, since the liquid permeability of the nonwoven fabric can be increased by the cotton with a fiber length of 20 mm or greater while the bulk of the nonwoven fabric can be increased by the cotton with a fiber length of 10 mm or smaller.

The content of cotton in the first fiber layer is not particularly restricted, but from the viewpoint of water absorption, water retention, flexibility and the like, it is, for example, in the range of 1 to 70 mass% and preferably in the range of 2 to 30 mass%.

Moreover, the cotton in the first fiber layer preferably contains the cotton fiber mass in the first fiber layer, and most preferably the cotton fiber mass is dispersed in a matrix composed of aggregates of the thermoplastic resin fibers. If the first fiber layer situated on the second surface side (non-skin-facing side) of the top sheet includes a cotton fiber mass, then when excreted fluid such as urine that has been absorbed and held in the absorbent body has been discharged from the absorbent body as moisture, the moisture can be absorbed and held in a concentrated (spot-like) manner in the cotton fiber mass in the first fiber layer, and the area of the sections where moisture has been absorbed and held in the in-plane direction of the first fiber layer (to a spot-like form), therefore making it possible to further minimize the amount of moisture discharged from the first surface side of the first fiber layer. As a result, moisture that has been discharged from the absorbent body can be effectively confined in the empty spaces of the protrusions of the nonwoven fabric, as described below.

According to the invention, the thermoplastic resin fibers in the first fiber layer are not particularly restricted so long as they are fibers made of a thermoplastic resin, and examples for the thermoplastic resin include publicly known resins including olefin-based resins such as polyethylene (PE), polypropylene (PP) and ethylene-vinyl acetate copolymer (EVA), polyester-based resins such as polyethylene terephthalate (PET) and polylactic acid (PLA), and polyamide-based resins such as 6-nylon, any of which resins may be used alone or as combinations of two or more of such resins.

Also, the structure of the fibers made of such thermoplastic resins is not particularly restricted, and examples include composite fibers such as core-sheath fibers, side-by-side fibers and island/sea fibers; hollow type fibers; modified cross-section fibers such as flat, Y-shaped or C-shaped fibers; solid crimped fibers with latent crimping or developed crimping; and splittable fibers that split by physical load such as a water stream, heat or embossing, and fibers having such structures may be used alone, or two or more different types may be used in combination.

The fineness of the thermoplastic resin fibers is not particularly restricted, but from the viewpoint of nonwoven fabric strength and flexibility, feel on the skin and liquid permeability, it will usually be in the range of 1.1 to 8.8 dtex and is preferably in the range of 1.5 to 4.6 dtex. Also, the fineness of the thermoplastic resin fibers in the first fiber layer is preferably smaller than the fiber size (fineness) of the hydrophobic thermoplastic resin fibers in the second fiber layer, described below. If the thermoplastic resin fibers in the first fiber layer have a smaller fiber size (fineness) than the hydrophobic thermoplastic resin fibers in the second fiber layer, then the thermoplastic resin fibers with a small fiber size in the first fiber layer will readily become entangled with the cotton in the first fiber layer and the thermoplastic resin fibers in the second fiber layer, and tearing of the fiber layer or interlayer separation between the fiber layers, that occurs due to dissociation between the cotton and thermoplastic resin fibers, will be less likely to occur, thus allowing the nonwoven fabric to maintain excellent strength even when the nonwoven fabric includes cotton as a constituent component.

The fiber lengths of the thermoplastic resin fibers in the first fiber layer are not particularly restricted, but from the viewpoint of nonwoven fabric strength, flexibility and liquid permeability, they are usually in the range of 20 to 100 mm and are preferably in the range of 35 to 65 mm. The thermoplastic resin fibers may be subjected to hydrophilicizing treatment, such hydrophilicizing treatment being, for example, treatment utilizing a surfactant, hydrophilic agent or the like (for example, kneading of a surfactant into the fiber interiors, or coating of the fiber surfaces with a surfactant).

The content of thermoplastic resin fibers in the first fiber layer is not particularly restricted, but from the viewpoint of nonwoven fabric strength, flexibility and the like, it is, for example, in the range of 30 to 99 mass% and preferably in the range of 70 to 98 mass%.

The first fiber layer may also include fibers other than the cotton and thermoplastic resin fibers, or optional additives, within a range such that the effect of the invention is not inhibited.

The second fiber layer of the fiber laminate used in the top sheet of the absorbent article of the invention will now be described. The second fiber layer is composed of hydrophobic thermoplastic resin fibers, the hydrophobic thermoplastic resin fibers being not particularly restricted so long as they have hydrophobicity, and any desired thermoplastic resin fibers may be used. If the second fiber layer forming the skin-facing surface (first surface) of the top sheet is formed by hydrophobic thermoplastic resin fibers, then the second fiber layer will be less prone to absorb and retain excreted fluid and moisture, thus helping to avoid the perception of a condition of dampness, or production of discomfort, for the wearer.

The term "hydrophobic" as used here means a property of being poorly compatible with water or resistant to retention of moisture, and for example, it corresponds to a contact angle of about 80° to 100° with ion-exchanged water. The contact angle with ion-exchanged water may be measured by the method described in "Measurement of initial contact angle" in Japanese Unexamined Patent Publication No. 2005-324010.

Moreover, when the fiber laminate is composed of two fiber layers consisting of a first fiber layer and a second fiber layer it is preferred, for the hydrophobic thermoplastic resin fibers composing the second fiber layer, to use the same type of fibers as the thermoplastic resin fibers in the first fiber layer (that is, fibers of the same material as the thermoplastic resin fibers in the first fiber layer, and similar fiber lengths), from the viewpoint of easier entanglement between the fibers.

The second fiber layer may also include fibers other than the thermoplastic resin fibers, or optional additives, within a range such that the effect of the invention is not inhibited.

According to the invention, the fiber laminate used as the top sheet of the absorbent article is not limited to a two-layer laminate as in the embodiment described above, and may be a laminate composed of three or more fiber layers, depending on the desired absorption properties and flexibility. Moreover, in the top sheet 2 of this embodiment, the first surface 2a (upper side) of the top sheet 2 is formed by a second fiber layer 202 composed of hydrophobic thermoplastic resin fibers 205. If the first surface of the top sheet contacting with the skin of the wearer of the absorbent article is formed by a second fiber layer containing no cotton or hydrophilic fibers, then excreted fluid such as urine that has been absorbed and held in the cotton of the first fiber layer will be less likely to contact with the skin of the wearer, and the absorbent article of the invention will be less prone to cause the perception of a condition of dampness for the wearer, or to produce discomfort for the wearer. In addition, if the first surface of the top sheet is formed of a fiber layer containing no cotton, this can prevent impairment of the feel on the skin or reduction in flexibility due to the cotton, and can help maintain an excellent feel on the skin and flexibility as a nonwoven fabric.

Moreover, in the fiber laminate described above, there are no particular restrictions on fiber layers other than the first fiber layer and the second fiber layer, and examples include fiber layers made of natural fibers such as wool, regenerated fibers such as rayon, inorganic fibers, and synthetic resin fibers such as thermoplastic resin fibers. Other fiber layers may also include optional additives, within a range such that the effect of the invention is not inhibited.

According to the invention, the basis weight of each fiber layer of the fiber laminate used in the top sheet is not particularly restricted, but from the viewpoint of strength, flexibility and absorption properties of the nonwoven fabric, each is preferably in the range of, for example, 1 to 60 g/m$^2$, and preferably in the range of 10 to 30 g/m$^2$. There are no particular restrictions on the basis weight of the nonwoven fabric, but it will usually be in the range of 10 to 100 g/m$^2$, preferably in the range of 15 to 75 g/m$^2$ and more preferably in the range of 20 to 50 g/m$^2$. The thickness of the fiber laminate is also not particularly restricted, but will usually be in the range of 0.1 to 5 mm, preferably in the range of 0.5 to 3 mm and more preferably in the range of 0.8 to 2 mm.

A method for producing a fiber laminate, prior to protrusions and recesses shaping to be used as the top sheet of an absorbent article of the invention, will now be described.

According to the invention, the means for producing the fiber laminate is not particularly restricted, and for example, it may be a method of using fibers for formation of each fiber layer (that is, cotton and thermoplastic resin fibers for formation of the first fiber layer and thermoplastic resin fibers for formation of the second fiber layer) to form a web (fleece) corresponding to each fiber layer, and physically or chemically bonding together the fibers in each web or between the webs.

Specifically, the following procedure may be followed to produce a two-layer fiber laminate including the aforementioned first fiber layer and second fiber layer.

(1) A production apparatus is prepared comprising a conveying apparatus for conveying of a sheet member in one direction, a first stage carding apparatus situated above the conveying apparatus and upstream in the machine direction, a second stage carding apparatus situated above the conveying apparatus and downstream in the machine direction (that is, downstream from the first stage carding apparatus), and an air-through system heating apparatus situated downstream from the second stage carding apparatus.

(2) Thermoplastic resin fibers for formation of the second fiber layer are supplied to the first stage carding apparatus, and the thermoplastic resin fibers are opened by the pins of a revolving roll in the carding apparatus, to form a web corresponding to the second fiber layer on the conveying surface of the conveying apparatus.

(3) Cotton and thermoplastic resin fibers for formation of the first fiber layer are supplied to the second stage carding apparatus while the web corresponding to the second fiber layer formed on the conveying surface of the conveying apparatus is being conveyed downstream in the machine direction, and the fibers are opened by the pins of a revolving roll in the carding apparatus to form a web corresponding to the first fiber layer, on the web corresponding to the second fiber layer while it is being conveyed.

(4) The layered web obtained by layering the web corresponding to the first fiber layer on the web corresponding to the second fiber layer is conveyed to the air-through system heating apparatus, and in the heating apparatus, the fibers within the web and between the webs are entangled, and the thermoplastic resin fibers may be optionally heat-fused together while fusing and anchoring the cotton on the front surfaces of the thermoplastic resin fibers, to produce a two-layer fiber laminate comprising the layered first fiber layer and second fiber layer.

When a fiber laminate comprising three or more fiber layers is to be produced, a production apparatus having three or more stages of carding apparatuses is provided and production is carried out by the same procedure described above.

The method of forming the web corresponding to each fiber layer is not limited to the method described above, and a wet method, for example, may be used. The method of bonding the webs is also not restricted to the method described above, and a hydroentangling method or needle punching method, for example, may be employed.

The fiber laminate produced in this manner is subjected to protrusions and recesses shaping to impart a specific structure composed of protrusions and recesses, and a fiber laminate having a structure composed of protrusions and recesses is obtained for use as the top sheet of an absorbent article of the invention.

The structure composed of protrusions and recesses of the top sheet to be used in the absorbent article of the invention will now be explained in greater detail with reference to the accompanying drawings.

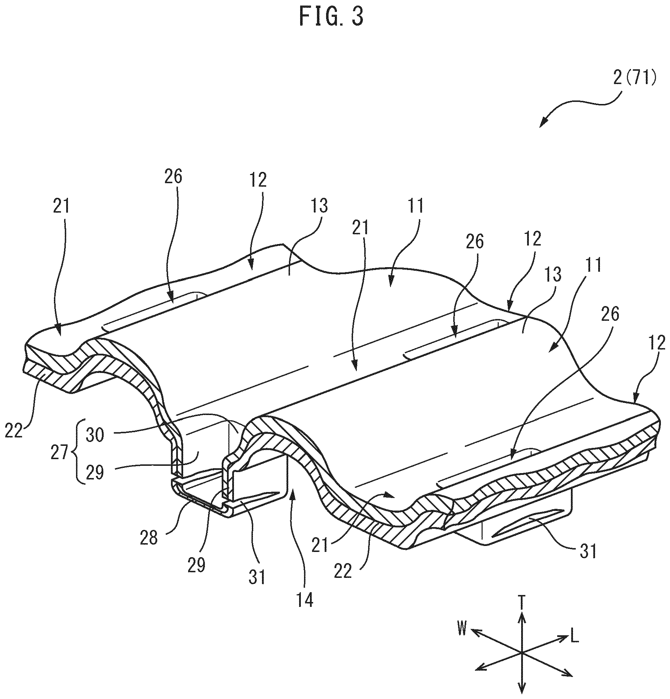

As shown in Figs. 3 to 7, the top sheet 2 comprises a first surface 2a located on the opposite side from the absorbent body 4 and a second surface 2b on the absorbent body side as the side opposite the first surface 2a, and the top sheet 2 also comprises a plurality of protrusions 11 protruding toward the first surface 2a, that are laid out in the lengthwise direction L and formed at a predetermined intervals in the widthwise direction W, and a plurality of recesses 12 depressed toward the second surface 2b, that are laid out in the lengthwise direction L and formed between the protrusions 11, while having empty spaces 14 on the second surface sides of the protrusions 11, faced by the second surface 2b of the top sheet 2 (nonwoven fabric).

In other words, as mentioned above, the top sheet 2 is a nonwoven fabric comprising a two-layer fiber laminate 200 that includes a first fiber layer 201 composed of cotton and thermoplastic resin fibers, and a second fiber layer 202 composed of hydrophobic thermoplastic resin fibers, wherein the nonwoven fabric is provided with a plurality of the protrusions 11 protruding toward the first surface side and a plurality of the recesses 12 depressed toward the second surface side, the protrusions 11 each having empty space 14 faced by the second surface 2b of the nonwoven fabric, and therefore even when excreted fluid such as urine from a wearer that has been absorbed and held in the absorbent body 4 has been discharged from the absorbent body 4 as moisture by evaporation and the like, to the side facing the skin of the wearer, the moisture is absorbed and held in the cotton in the first fiber layer 201 while being retained in a state of moisture in the empty spaces 14 (i.e. the empty spaces 14 are in a highly humid state), such that a state of gas-liquid equilibrium is formed between the moisture in the empty spaces 14 (the gas phase) and the excreted fluid absorbed and held in the absorbent body 4 (the liquid phase), and further discharge of moisture from the absorbent body 4 can be inhibited.

Thus, the disposable diaper 1 of this embodiment is less prone to produce a feeling of mustiness or the like for the wearer or to create discomfort for the wearer.

Also, as shown in Figs. 3 to 7, according to this embodiment the recesses 12 each have a first recess 21 comprising a first bottom section 22 located further in the absorbent body 4 side than the location of the first surface 2a at the top sections 13 of the protrusions 11, and a plurality of second recesses 26 provided in a discontinuous manner in the lengthwise direction in the first recess 21, and formed as depressions opening into the first bottom section 22. Also, each second recess 26 comprises perimeter wall sections 27 extending in the direction from the first bottom section 22 to the absorbent body 4, and a second bottom section 28 having the highest fiber density of the top sheet 2, provided at the edges of the perimeter wall sections 27 on the absorbent body 4 sides, so as to plug the edges.

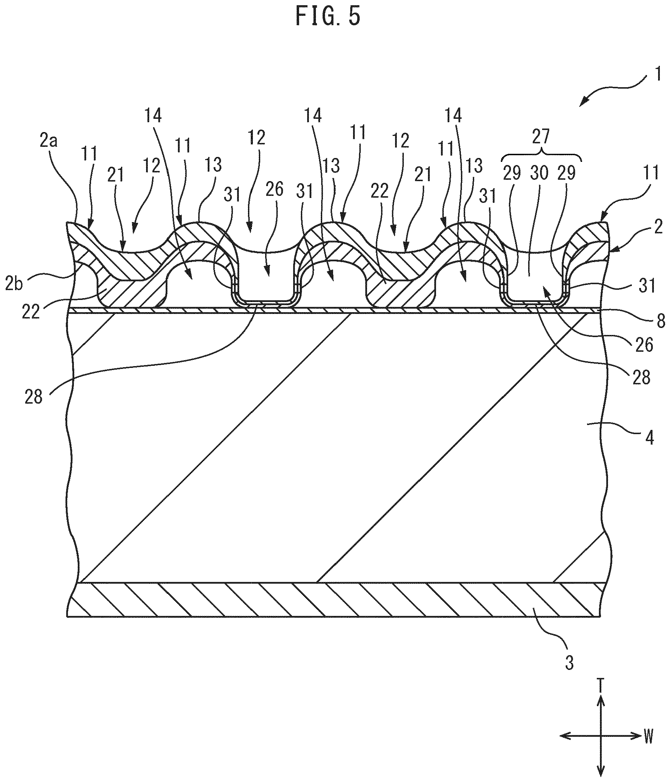

Furthermore, in the top sheet 2 of this embodiment, the second surface 2b is not joined to the absorbent body 4 at the top sections 13 of the protrusions 11, but the second bottom sections 28 of the second recesses 26 of the recesses 12 are joined to the sections of the top sheet side of the absorbent body 4 (the surface of the top sheet side of the absorbent body 4, according to this embodiment) by an adhesive layer 8. Also, as shown in Figs. 5 to 7, for this embodiment, portions of the second surfaces 2b of the first bottom sections 22 of the first recesses 21 are also joined to portions of the top sheet side of the absorbent body 4 by an adhesive layer 8. At the protrusions 11, on the other hand, portions of the second surfaces 2b including the top sections 13 are not joined to the absorbent body 4.

As shown in Fig. 3, Fig. 5 and Fig. 6, the top sheet 2 of this embodiment has a construction in which the regions of the first surface 2a and the regions of the second surface 2b forming the protrusions 11 are curved into convex shapes in the direction from the second surface 2b to the first surface 2a (i.e. in the direction opposite to the direction toward the absorbent body 4). On the other hand, the first recesses 21 of the recesses 12 have a construction in which the regions of the first surface 2a and the regions of the second surface 2b forming the first recesses 21 are curved into convex shapes in the direction from the first surface 2a side to the second surface 2b side (i.e. in the direction toward the absorbent body 4). The top sheet 2 therefore has an essentially wavy cross-section with alternately repeating protrusions and recesses with respect to the widthwise direction W.

The protrusions 11 are laid out in the lengthwise direction of the surface (sheet surface) of the disposable diaper 1 (that is, the lengthwise direction of the top sheet 2), disposed in a plurality of rows at predetermined intervals in the widthwise direction of the top sheet 2. For this embodiment, each of the protrusions 11 is laid out continuously in the lengthwise direction L, so as to be mutually substantially parallel with the other protrusions 11.

Also, although the protrusions 11 are laid out even beyond both edges in the lengthwise direction (first direction) of the absorbent body in this embodiment, there is no limitation to this mode for the invention, and the protrusions may be laid out either up to at least one of the edges or beyond one of the edges, in the first direction of the absorbent body. By providing the protrusions in this manner, not only moisture discharged from the skin-facing side of the absorbent body, but also moisture discharged from the edges (side surfaces) in the lengthwise direction (first direction) of the absorbent body can be confined to the empty spaces on the second surface sides of the protrusions, and thus further help to protect the wearer from a feeling of mustiness or the like.

The protrusions according to the invention have intervals with the other adjacent protrusions of preferably 0.25 to 5 mm, more preferably 0.5 to 3 mm and even more preferably 0.75 to 2 mm. Here, the intervals between adjacent protrusions are the distances between approximately the center locations of the respective protrusions in the widthwise direction of the top sheet (essentially the top sections of the protrusions). If the distance of the interval between adjacent protrusions is less than 0.25 mm, the structure composed of protrusions and recesses of the nonwoven fabric will be too narrow and it will not be possible to significantly reduce the contact area between the protrusions of the nonwoven fabric and the skin of the wearer, potentially impairing the feel on the skin of the front surface (first surface) of the nonwoven fabric, while if the distance of the interval between adjacent protrusions is greater than 5 mm, the difference in structure compared to the nonwoven fabric (fiber laminate) before protrusions and recesses shaping will be less prominent, making it difficult to obtain a flexible feel on the skin by implementation of the structure composed of protrusions and recesses.

Moreover, according to the invention, the protrusions have lengths from the heights on the first surface sides of the first bottom sections of the first recesses to the heights of the top sections of the protrusions (i.e. heights of the protrusions) of preferably 0.25 to 5 mm, more preferably 0.5 to 3 mm and even more preferably 0.75 to 2 mm. If the heights of the protrusions are less than 0.25 mm, the size of the protrusions will be too small, making it impossible to obtain a flexible feel on the skin by implementation of the structure composed of protrusions and recesses, while if the heights of the protrusions are greater than 5 mm, the protrusions will excessively protrude creating a sharp structure, such that it will be difficult to obtain a flexible feel on the skin.

Also, as shown in Fig. 5, the distance between the sections of the second surfaces 2b at the top sections 13 of the protrusions 11, and the top sheet side sections of the absorbent body 4 is smaller than the distance between the sections of the first surfaces 2a of the first bottom sections 22 nearest the absorbent body 4 and the top sheet side section of the absorbent body 4. According to this aspect, therefore, the top sections 13 of the protrusions 11 are disposed, overall, at locations more distant from the top sheet 2 (the upper side in Fig. 5) than the first surfaces 2a of the first bottom sections 22. Since spaces are thus stably formed as empty spaces 14 between the second surfaces 2b of the protrusions 11 and the top sheet side surface of the absorbent body 4, even if the protrusions 11 of the top sheet 2 are subjected to external force from the wearer, and particularly shear force in the widthwise direction accompanying friction with the skin of the wearer, the protrusions 11 deform or collapse, depending on the direction and degree of the external force, so that the force is easily absorbed. Thus, tensile force of the top sheet 2 generated by friction between the top sheet 2 and the skin of the wearer is reduced, and detachment from the absorbent body 4 can be more reliably avoided.

Furthermore, since the top sheet 2 is composed of a nonwoven fabric comprising a two-layer fiber laminate 200 that includes a first fiber layer 201 composed of cotton and thermoplastic resin fibers, and a second fiber layer 202 composed of hydrophobic thermoplastic resin fibers, as mentioned above, even when excreted fluid from a wearer that has been absorbed and held in the absorbent body 4 has been discharged from the absorbent body 4 as moisture to the side facing the skin of the wearer, the moisture is absorbed and held in the cotton in the first fiber layer 201 while being retained in a state of moisture in the empty spaces 14, such that a state of gas-liquid equilibrium is formed between the moisture in the empty spaces 14 (the gas phase) and the excreted fluid absorbed and held in the absorbent body 4 (the liquid phase), and further discharge of moisture from the absorbent body 4 can be inhibited.

For this embodiment, the first recesses 21 are formed integrally with the protrusions 11 in the widthwise direction. Also, the first bottom sections 22 of the first recesses 21 have the greatest maximum thickness among the thickness of the top sheet 2, and the first bottom sections 22 are sections with excellent elasticity as a whole. The plurality of first recesses 21 formed in the top sheet 2 are all formed with mutually equal widths.

For this embodiment, the second surfaces 2b of the first bottom sections 22 of the first recesses 21 are bonded to the top sheet side section of the absorbent body 4, at least at the sections of the first bottom sections 22 located furthest to the top sheet side, i.e. the sections located at a depth most distant from the locations of the heights of the top sections 13 of the protrusions 11.

For this embodiment, the second recesses 26 have essentially rectangular openings in a plane view (as viewed from the side of the first surfaces 2a of the top sheet 2 in the thickness direction), and protrude overall to the absorbent body 4 side of the top sheet 2, defining essentially cuboid interior spaces. Also, the second recesses 26 are disposed at a constant interval in the lengthwise direction of each of the recesses 12 (that is, the lengthwise direction of the first recesses 21), each of the second recesses 26 being formed in a mutually independent manner from the other second recesses 26.

For this embodiment, the perimeter wall sections 27 comprise a pair of first perimeter wall sections 29, 29 formed along the lengthwise direction of the top sheet 2 and a pair of second perimeter wall sections 30, 30 formed along the widthwise direction of the top sheet 2, the pair of first perimeter wall sections 29, 29 being mutually disposed facing each other, and the pair of second perimeter wall sections 30, 30 also be mutually disposed facing each other.

Also, as shown in Fig. 3, Fig. 5 and Fig. 6, the pair of first perimeter wall sections 29, 29 have holes 31 formed therein running from the interior spaces of the first recesses 21 to the second surfaces 2b. According to this embodiment, one hole 31 is provided for each of the pair of first perimeter wall sections 29, 29, and the holes 31 are formed at locations near the second bottom sections 28 of the first perimeter wall sections 29 (and therefore two holes 31 are present for each first recess 21). On the other hand, the pair of second perimeter wall sections 30, 30 does not have corresponding holes 31, each of the second perimeter wall sections 30 having its entire edge on the absorbent body 4 side directly connected to the second bottom section 28.

One reason that the second recesses 26 are provided in the top sheet 2 is in order to reduce to a minimum the likelihood that the bottoms of the recesses 12, and more specifically the first bottom sections 22 of the first recesses 21, will contact the skin of the wearer, and in order to absolutely minimize the contact area even when the first bottom sections 22 have contacted with the skin of the wearer. Specifically, the top sheet of the invention has a construction wherein the skin of the wearer is most easily contacted with first the protrusions and then the first bottom sections of the first recesses, having a preferred structure wherein the protrusions with the highest flexibility of the top sheet, that are not bonded to the absorbent body, contact more easily with the skin of the wearer than the first bottom sections, but also according to the invention, considering that a smaller contact area contacted with the skin of the wearer is more likely to provide a feeling of flexibility, there are formed sections provided with the second recesses and lacking the first recesses, to further reduce the sections of the first bottom sections of the first recesses contacting with the skin of the wearer and reduce to a minimum both the opportunity and contact area of contact with the skin.

Furthermore, according to the invention, the second recesses are provided on the first bottom sections of the first recesses in order to help prevent an uncomfortable feeling or sensation of foreign matter by contact with the second recesses, the second recesses serving to reduce a minimum the likelihood of contact with the skin of the wearer.

Furthermore, the top sheet used in the absorbent article of the invention comprises a structure having the aforementioned specified protrusions and recesses, i.e. a structure wherein the recesses are first recesses comprising first bottom sections located more toward the second surface side than the location of the first surface side at the top sections of the protrusions, and second recesses comprising perimeter wall sections extending from the first bottom sections toward the second surface side and second bottom sections formed at the edges of the second surface sides of the perimeter wall section so as to plug the edges, wherein stress applied in the thickness direction from the first surface side of the top sheet can be buffered by deformation of the first recesses toward the second surface side, thereby allowing sufficient flexibility to be ensured even when the nonwoven fabric composing the top sheet includes cotton.

According to the invention, the depths of the second recesses, i.e. the lengths from the heights on the first surface (upper side) of the first bottom sections of the first recesses to the heights on the first surface side of the second bottom sections of the second recesses, are preferably 0.05 to 2 mm, more preferably 0.075 to 1.5 mm and even more preferably 0.1 to 1 mm. If the depths of the second recesses are less than 0.05 mm, it will be difficult to ensure rigidity of the second bottom sections as described below, and the strength of the nonwoven fabric in the thickness direction will tend to be insufficient. If the depths of the second recesses are greater than 2 mm, conversely, the strength in thickness direction will tend to be insufficient when the other members (such as the absorbent body, nonwoven fabric or film) of the absorbent article are attached, and a hard feel may result under compression.

Furthermore, the relationship between the depths of the second recesses and the heights of the protrusions is such that the depths of the second recesses (that is, the lengths from the heights on the first surface sides of the first bottom sections of the first recesses to the heights on the first surface sides of the second bottom sections of the second recesses) is preferably 10 to 80%, more preferably 15 to 70% and more preferably 20 to 60% of the heights of the protrusions (that is, the lengths from the heights on the first surface sides of the first bottom sections of the first recesses to the heights of the top sections of the protrusions). If the depths of the second recesses are less than 10% of the heights of the protrusions, it will not be possible to sufficiently ensure the spaces formed by the holes at the perimeter wall sections of the second recesses, resulting in insufficient or lack of formation of the holes and tending to prevent excellent flexibility as a nonwoven fabric. Conversely, if the depths of the second recesses exceed 80% of the heights of the protrusions, the holes will be formed excessively large, such that the strength of the perimeter wall sections of the second recesses will be lower tending to result in napping, and potentially resulting in a poorer feel on the skin of the nonwoven fabric.

According to the invention, there is no particular restriction on the lengths in the first direction (lengthwise direction) of the second recesses, but they are preferably 0.25 to 5 mm, more preferably 0.5 to 3 mm and even more preferably 0.75 to 2 mm. If the lengths of the second recesses in the first direction are less than 0.25 mm, the second recesses will be too small and it may not be possible to adequately obtain the aforementioned effect exhibited by the second recesses. If the lengths of the second recesses in the first direction exceed 5 mm, then the second recesses will be too long in the first direction, and it will be difficult to distinguish flexibility from that of a flat nonwoven fabric or a nonwoven fabric without recesses, and it will be difficult to obtain a flexible feel on the skin.

There is also no particular restriction on the lengths of the second recesses in the second direction (widthwise direction), but they are preferably 0.25 to 5 mm, more preferably 0.5 to 3 mm and even more preferably 0.75 to 2 mm.

If the lengths of the second recesses in the second direction are less than 0.25 mm, the second recesses will be too small, and formation of the second bottom sections in particular will be insufficient, potentially making it impossible to adequately obtain the aforementioned effect exhibited by the second recesses. If the lengths of the second recesses in the second direction are greater than 5 mm, the second recesses will become too large, potentially causing an uncomfortable feeling or sensation of foreign matter upon contact with the nonwoven fabric.

Also, the pitch of the second recesses in the second direction (that is, the distance between adjacent second recesses in the second direction) is not particularly restricted, but is preferably no greater than 2.0 mm and more preferably no greater than 1.5 mm. If the pitch of the second recesses in the second direction is 2.0 mm or smaller, cotton in the first fiber layer composing the nonwoven fabric will be easily held in the second bottom sections of the second recesses, thus helping to further prevent splitting of the fiber layer by dissociation between the cotton and thermoplastic resin fibers in the first fiber layer, or interlayer separation between the fiber layers, at the second bottom sections. In addition, if the pitch is 2.0 mm or smaller, excreted fluid such as urine that has been supplied from the first surface side of the nonwoven fabric will be easily drawn in a spot-like manner into the second bottom sections of the second recesses disposed between adjacent protrusions, thereby allowing an even more excellent absorption property to be exhibited as a top sheet (especially absorption rate and liquid migration), while the moisture that has been discharged from the absorbent body will be even less prone to migrate from the second surface side of the nonwoven fabric to the first surface side. As a result, moisture that has been discharged from the absorbent body can be effectively confined in the empty spaces of the protrusions, and the wearer can be even further protected from feeling mustiness and the like.

Furthermore, for this embodiment, holes 31 are provided in each of the pair of first perimeter wall sections 29, 29 of the second recesses. One reason for providing the holes 31 on the first perimeter wall sections 29 is in order to release the tensile force of the fibers of the recesses 12 provided with the holes 31 and of the adjacent protrusions 11, increasing the freedom of movement of the protrusions 11 as a whole or of the fibers forming the protrusions 11, and to improve the flexibility of the protrusions 11, and more specifically the flexibility of the protrusions 11 in the thickness direction of the top sheet 2, as well as the flexibility in the lengthwise direction (first direction) or widthwise direction (especially the widthwise direction) of the top sheet 2 when it slides against the skin, to ensure a smooth feel. This allows the protrusions 11 to be imparted with both an excellent hard/soft feeling (excellent softness in the thickness direction) and an excellent rough/smooth feeling in the lengthwise direction and widthwise direction (excellent smoothness on the front surface of the top sheet 2 (particularly the widthwise direction)), to allow an excellent hard/soft feeling and rough/smooth feeling to be ensured for the top sheet 2 as a whole, while also allowing a flexible feel on the skin to be achieved.

On the other hand, one reason for not providing holes 31 on the second perimeter wall sections 30 is in order to help prevent the level differences, created by the second recesses 26, from catching on the skin when the skin slides in the lengthwise direction of the top sheet 2 (the first direction), i.e. in the direction in which the protrusions 11 or recesses 12 extend, to ensure smoothness in the lengthwise direction of the top sheet 2. That is, since the second perimeter wall sections 30 are continuous with the first bottom sections 22 and second bottom sections 28 and integral without seams, the skin does not significantly sense the level difference of the first bottom sections 22 in the second recesses 26 and tends to smoothly move along the protrusions 11 and recesses 12 when the skin slides in the lengthwise direction of the top sheet 2. This can ensure smoothness in the lengthwise direction of the top sheet 2 due to the flexibility of the protrusions 11 or flexibility of the fibers.

Moreover, one reason for providing the holes 31 at locations of the first perimeter wall sections 29 near the second bottom sections 28 is in order for the holes 31 to be as far as possible from the protrusions 11 or first bottom sections 22 that tend to contact the skin, thereby minimizing opportunity for the holes 31 to contact the skin of the wearer and helping to reduce any uncomfortable feeling or sensation of foreign matter. This can more stably ensure smoothness when the skin slides in the planar direction of the nonwoven fabric.

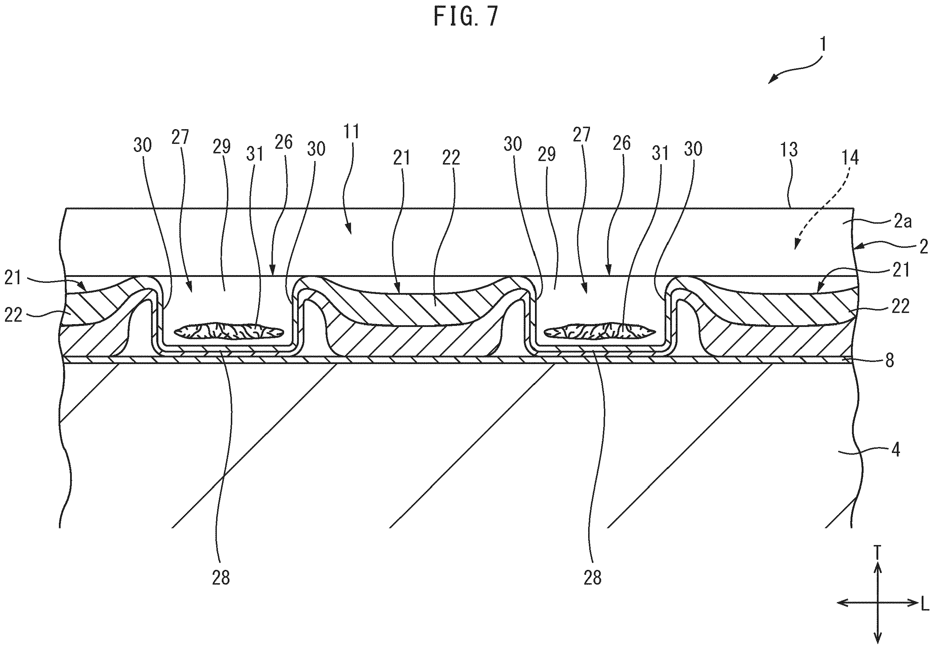

Furthermore, as shown in Fig. 7, the holes 31 are formed by breaking the thermoplastic resin fibers contained in the top sheet 2 and not by melting of the thermoplastic resin fibers, and the perimeters of the holes 31 include, among the thermoplastic resin fibers in the nonwoven fabric composing the top sheet 2, the broken ends of broken fibers having broken ends formed by their breakage. The broken ends of the broken fibers are ends formed by breaking, accomplished by pulling or physical cutting in the lengthwise directions of the thermoplastic resin fibers, and instead of being melted and rounded fiber ends with increased fiber sizes, as when thermoplastic resin fibers have melted, the broken ends are in a tapered state formed by tearing of the fibers, or else are in a state with virtually no change in fiber size. Thus, even when the skin of the wearer has been contacted with the perimeters of the holes 31, the absence of thermoplastic resin fibers hardened by melting helps to suppress any uncomfortable feeling due to stiffness or catching of the fibers, while also minimizing any feeling of hardness or roughness from the top sheet 2.

Furthermore, as shown in Fig. 7, some of the fibers among the thermoplastic resin fibers pass through the interior spaces of the holes 31, with some of the broken fibers having their broken ends protruding into the interior spaces of the holes 31. That is, the interior spaces of the holes 31 have a combination of thermoplastic resin fibers passing through the interior spaces and thermoplastic resin fibers extending into the interior spaces, so that the spaces are not completely open. Thus, since the holes 31 have some of the thermoplastic resin fibers passing through, or extending into, the interior spaces, even if the skin of the wearer has contacted the holes 31, the thermoplastic resin fibers in the interior spaces can reduce the level differences between the first perimeter wall sections 29 or the second bottom sections 28 of the second recesses 26, and the holes 31, to minimize any difference in touch sensation and to reduce any uncomfortable feeling for the wearer contacted.

Incidentally, the open area percentage of the interior spaces of the holes is preferably 1 to 50%, more preferably 1.5 to 35% and even more preferably 2.5 to 20%. If the open area percentage of the interior spaces of the holes is less than 1%, the open area percentage will be too low, making it impossible to impart freedom to the protrusions or the fibers of the protrusions, or to adequately ensure flexibility for the protrusions. If the open area percentage of the interior spaces of the holes is greater than 50%, conversely, the strength of the first perimeter wall sections in which the holes are formed will tend to be reduced, and the borders of the perimeters of the holes may tend to be more easily felt. However, the open area percentage of the interior spaces of the holes may be outside of this range, and set as desired, depending on the type of absorbent article and its purpose of use, etc.

In addition, for this embodiment, in the second bottom sections 28, the nonwoven fabric composing the top sheet 2 is formed by being compacted in the direction from the first surfaces 2a to the second surfaces 2b, and they have the highest fiber density of the top sheet 2, as mentioned above, as well as the highest rigidity. As a result, in its thickness direction, the nonwoven fabric composing the top sheet 2 has a density gradient from the protrusions with relatively low fiber density to the second bottom sections of the second recesses having the highest fiber density, such that excreted fluid such as urine supplied from the first surface side of the nonwoven fabric is easily drawn in a spot-like manner from the protrusions to the second bottom sections of the second recesses, allowing excellent absorption properties (especially absorption rate and liquid migration properties) to be exhibited as a top sheet, and helping to prevent moisture that has been discharged from the absorbent body, from migrating from the second surface side to the first surface side of the nonwoven fabric, as mentioned above. As a result, moisture that has been discharged from the absorbent body can be effectively confined in the empty spaces of the protrusions, and the wearer can be even further protected from a feeling mustiness or the like.

In addition, the first surfaces 2a of the second bottom sections 28 (i.e. the inner space side surface of the second recesses) and the second surfaces 2b (i.e. the absorbent body 4 side surface) are formed in an essentially planar manner overall. For this embodiment, the second surfaces 2b of the second bottom sections 28 are formed essentially flat, and are in contact with the top sheet side surface of the absorbent body 4. When the second surfaces 2b of the second bottom sections 28 are thus formed flat, it is possible to maximally increase the contact area between the second bottom sections 28 and the absorbent body 4, thereby making it possible to ensure that the joining regions are as large as possible between the second surfaces 2b of the second bottom sections 28 and the surfaces on the top sheet side of the absorbent body 4. As a result, the top sheet 2 is even less likely to detach from the absorbent body 4, and migration of excreted fluids between the top sheet 2 and the absorbent body 4 can proceed more smoothly. Since as mentioned above, particularly for this embodiment, the top sheet side surface of the absorbent body 4 is a flat surface, the second surfaces 2b of the second bottom sections 28 are mutually bonded with their surfaces in contact with the top sheet side surface of the absorbent body 4.

Thus, the top sheet 2 is even more stably joined with the absorbent body 4, rendering them less likely to become detached from each other, and migration of excreted fluids between the top sheet 2 and the absorbent body 4 can be accomplished very smoothly.

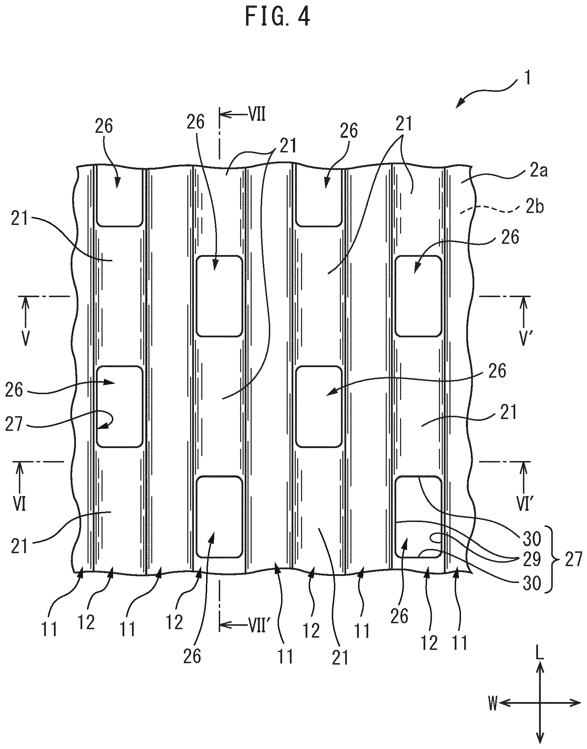

For this embodiment, as shown in Fig. 4, the second recesses 26 are disposed on the top sheet 2 in a zigzag fashion in a plane view. Also, as shown in Fig. 5, the top sheet 2 has a construction in which at least two protrusions 11 are situated between each second recess 26 and another second recess 26 adjacent to the second recess 26 in the widthwise direction. That is, the top sheet 2 has a construction with a protrusion 11, first recess 21 and a protrusion 11 disposed in that order, between each second recess 26 and its adjacent second recess 26 in the widthwise direction. One reason for constructing the top sheet 2 in this manner is in order to more stably minimize detachment of the top sheet 2 from the absorbent body 4, to maintain the flexibility of the protrusions. That is, since the top sheet 2 has the second recesses 26 joined to the absorbent body 4, joining of the second recesses 26 with the absorbent body can potentially create tensile force in the top sheet 2 in the widthwise direction, but by providing first bottom sections 22 that have lower fiber density than the second bottom sections 28 and deform more easily than the second bottom sections 28, between adjacent second bottom sections 28, 28, the tensile force can be absorbed by deformation of the first bottom sections 22. This allows the flexibility of the protrusions 11 to be stably maintained without interfering with freedom of the protrusions 11 in the widthwise direction.

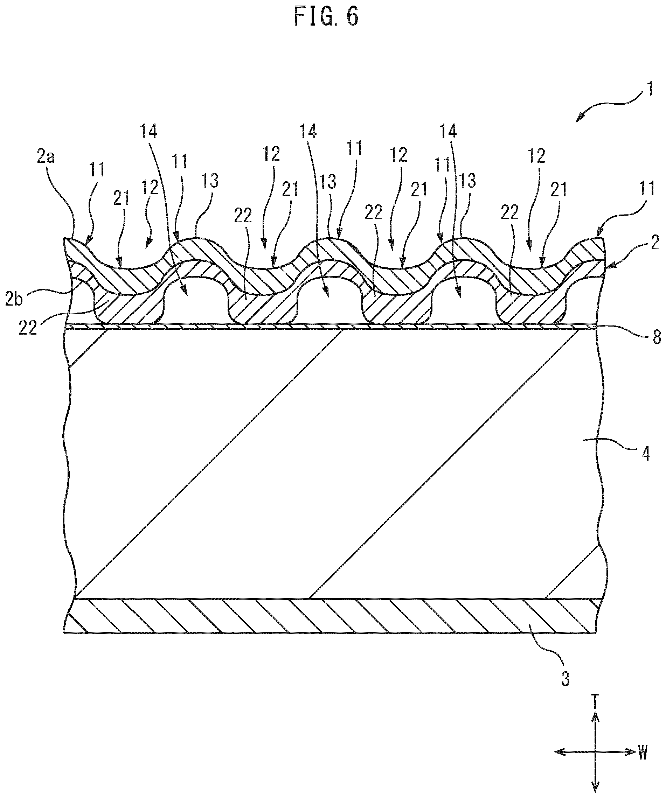

Furthermore, as shown in Fig. 6, in the top sheet 2, the construction in which first recesses 21 of the recesses 12 and the first recesses 21 of other recesses 12 adjacent to those recesses 12 are mutually adjacent has a portion that is continuous over the entire width in the widthwise direction. That is, the top sheet 2 has a construction with first recess 21, a protrusion 11 and first recess 21 disposed repeatedly in that order across the entire width in the widthwise direction, and a portion where no second recesses 26 are present. This is because in the top sheet 2, the second recesses 26 are disposed in a zigzag fashion while the intervals between the second recesses 26 in the lengthwise direction are set wider than the sizes of the second recesses 26 in the lengthwise direction.

One reason for constructing the top sheet 2 in this manner is in order to utilize the ease of deformation of the first bottom sections 22 in the first recesses 21 to impart higher flexibility to the protrusions 11. That is, if the top sheet 2 has a construction in which the first recesses 21 and protrusions 11 are alternately arranged across the entire width in the widthwise direction and no second recesses 26 joined to the absorbent body 4 are present between adjacent protrusions 11, then the first bottom sections 22 that have lower fiber density than the second bottom sections 28 and deform more easily than the second bottom sections 28 will be more easily able to have the protrusions 11 follow the surface of the skin as the wearer moves, and the protrusions can fit more stably onto the skin. Furthermore, since the flexibility of the first bottom sections 22 can be used to allow the protrusions 11 to also deform more flexibly, it is possible to ensure even higher flexibility for the top sheet 2 as a whole.

An example of a method for producing a disposable diaper 1 according to an embodiment of the invention will now be described.

In the method for producing a disposable diaper 1 according to this embodiment there are carried out, in order, a step of forming an absorbent body, a step of layering a top sheet web, formed by a step of forming a long top sheet web (that is, a web obtained by shaping a structure composed of protrusions and recesses in a fiber laminate (nonwoven fabric)) to serve as the top sheet described hereunder, onto an absorbent body, to form a laminate of the web of the nonwoven fabric for the top sheet and the absorbent body, and a step of optionally forming compressed grooves in the laminate. There are further carried out, in order, a step of layering a continuous sheet for the long back sheet, to serve as the back sheet, on the laminate to form a disposable diaper web, and a cutting step in which individual disposable diapers are cut from the disposable diaper web.

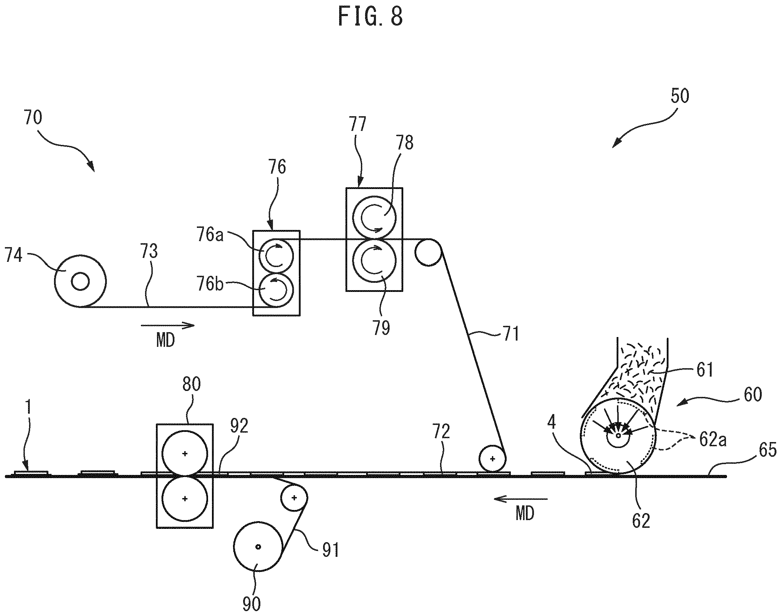

A production apparatus 50, such as shown in Fig. 8 to Fig. 11, for example, is used for production of the disposable diaper 1 of this embodiment. The production apparatus 50 comprises an absorbent body-forming apparatus 60 comprising a freely rotating suction drum 62 that forms the absorbent body 4 by layering an absorbent material 61, and a top sheet-forming apparatus 70 that forms a top sheet web 71 that is to serve as the top sheet 2 (that is, a nonwoven fabric web obtained by shaping a structure composed protrusions and recesses in the fiber laminate 200). It further comprises a back sheet roll 90 having a back sheet web 91 that is to serve as the back sheet 3 wound into a roll, and a cutting apparatus 80 that cuts the disposable diaper web 92 having the top sheet web 71 and back sheet web 91 mounted on the absorbent body 4, to form each individual disposable diaper 1.

The procedure for producing a disposable diaper 1 using this production apparatus 50 will now be explained in detail.

In the step of producing the absorbent body, at the absorbent body-forming apparatus 60, an absorbent body material 61 such as a super-absorbent polymer (SAP) is supplied from above onto the outer peripheral surface of the rotating suction drum 62, drawing the absorbent body material 61 into a depression-molding space 62a that is provided on the outer peripheral surface of the suction drum 62 and matches the shape of the absorbent body 4, and the absorbent body material 61 is accumulated to form an absorbent body 4. Next, the rotating suction drum 62 transfers and mounts the absorbent body 4 in the molding space 62a onto a carrier sheet 65 being conveyed in the machine direction MD under the suction drum 62. The carrier sheet 65 is conveyed downstream in the machine direction MD together with the mounted absorbent body 4, and is supplied to the following step.

The step of producing the absorbent body is followed by a step of forming a laminate. In this step, first the top sheet 2 is formed as a top sheet web 71 that is continuous in the lengthwise direction, by the top sheet-forming apparatus 70. Also, an adhesive layer comprising a hot-melt adhesive is formed on the first surface of the absorbent body 4 that is being conveyed in the machine direction MD (on the upper side surface of Fig. 8), and the top sheet web 71 laminate is layered and bonded over it, to obtain a laminate 72. The laminate 72, once formed, is conveyed downstream in the machine direction MD and supplied to the following step.

After the step of forming the laminate, the step of forming the disposable diaper web is carried out. In this step, the back sheet web 91 that has been reeled out from the back sheet roll 90 is successively layered onto the absorbent body 4 side surface of the laminate 72 (the lower side surface of the laminate 72 in this case), from the lower side of the absorbent body 4, via an adhesive layer comprising a hot-melt adhesive, to form a disposable diaper web 92 in which a plurality of disposable diapers 1 are directly connected in the lengthwise direction. This yields a disposable diaper web 92, wherein the top sheet web 71 is bonded to the first surface (upper side surface) of the absorbent body 4 while the back sheet web 91 is bonded to the surface on the side opposite the first surface (the lower side surface of the absorbent body 4 in this case). The disposable diaper web 92 is then conveyed downstream in the machine direction MD and supplied for the next step.

The cutting step is carried out after the step of forming the disposable diaper web. In this step, the cutter in the cutting apparatus 80 cuts individual disposable diapers 1 having prescribed shapes and sizes from the disposable diaper web 92. A disposable diaper 1 is thus obtained as a product.

The step of forming the top sheet web will now be explained, as part of the aforementioned step of forming the laminate.

In the step of forming the top sheet web, there are carried out in order a preheating step in which a nonwoven fabric 73 to be processed is unrolled from a top sheet roll 74 around which the long nonwoven fabric 73 to be processed (fiber laminate 200), serving as the starting material for the top sheet web 71, is wrapped as a roll, the nonwoven fabric 73 to be processed that has been unrolled is preheated, and a shaping step in which the nonwoven fabric 73 to be processed, that has passed through the preheating step, is stretched and shaped into a structure composed of protrusions and recesses. The long nonwoven fabric 73 that is to serve as the top sheet web 71 is the fiber laminate 200 obtained by the method for producing a fiber laminate prior to the structure composed of protrusions and recesses shaping step described above.

The step of forming the top sheet web is carried out using a top sheet-forming apparatus 70, such as illustrated in Fig. 8 to Fig. 11. The forming apparatus 70 comprises a top sheet roll 74 that rolls out the nonwoven fabric 73 to be processed downstream in the machine direction MD, a preheating apparatus 76 that applies preheating to the nonwoven fabric 73 to be processed that has been rolled out from the top sheet roll 74, and a shaping apparatus 77 that stretches the nonwoven fabric 73 to be processed that has been preheated, for a structure composed of protrusions and recesses shaping to form protrusions 11 and recesses 12 (including the first recesses 21 and second recesses 26).

In the preheating step, the long nonwoven fabric 73 to be processed, that has been rolled out from the top sheet roll 74 and conveyed along the machine direction MD, is successively contacted with the outer peripheral surfaces of a pair of rotating upper and lower heated rolls 76a, 76b of the preheating apparatus 76, to successively heat both sides surfaces of the nonwoven fabric 73 to be processed for preheating. Specifically, the nonwoven fabric 73 to be processed that has been rolled out from the top sheet roll 74 is first taken up onto the outer peripheral surface of the lower heated roll 76b, and the surface of the nonwoven fabric 73 to be processed in contact with the outer peripheral surface is heated. Next, the nonwoven fabric 73 to be processed is delivered to the upper heated roll 76a so that the surface opposite the surface heated by the lower heated roll 76b is contacted with the outer peripheral surface of the upper heated roll 76a, and the surface in contact with the outer peripheral surface is heated by the upper heated roll 76a.

The preheating temperature will depend on the type of thermoplastic resin fibers composing the nonwoven fabric to be processed, and for example, heating is preferably at or above the temperature of initial softening of the thermoplastic resin of the thermoplastic resin fibers used in the nonwoven fabric to be processed (the softening point), and a temperature below the melting point of the thermoplastic resin. If the preheating temperature is at or above the melting point, the thermoplastic resin fibers will harden after melting, thus potentially impairing the flexible feel of the top sheet on the skin. Also, if the preheating temperature is below the softening point of the thermoplastic resin, it will be difficult to shape the nonwoven fabric to be processed in the subsequent shaping step, and difficult to form a suitable structure composed of protrusions and recesses. For example, when the thermoplastic resin fibers in the nonwoven fabric to be processed are core-sheath composite fibers comprising polyethylene terephthalate (PET) and high-density polyethylene (HDPE), the thermoplastic resin composing the composite fibers will have a melting point temperature of 120°C and will begin to soften from about 50°C, such that the temperature of the outer peripheral surface of the heated roll is preferably set to about 50 to 110°C and more preferably about 60 to 100°C.

In the shaping step, the nonwoven fabric 73 to be processed, that has passed through the preheating step and been conveyed, is inserted between the engaged and rotating pair of upper and lower stretching rolls 78, 79 of the shaping apparatus 77, stretching the nonwoven fabric 73 to be processed between the ridges 78a and grooves 78b of the engaged upper stretching roll 78 and the outer peripheral surface sections 79b and pins 79a on the outer peripheral surface of the lower stretching roll 79, to shape it. To facilitate shaping when carrying out the shaping step, it is preferably carried out while heating the stretching rolls 78, 79. The heating temperature during this time is preferably a higher temperature than the preheating temperature during the preheating step, and a lower temperature than the melting point of the thermoplastic resin of the thermoplastic resin fibers in the nonwoven fabric 73 to be processed.

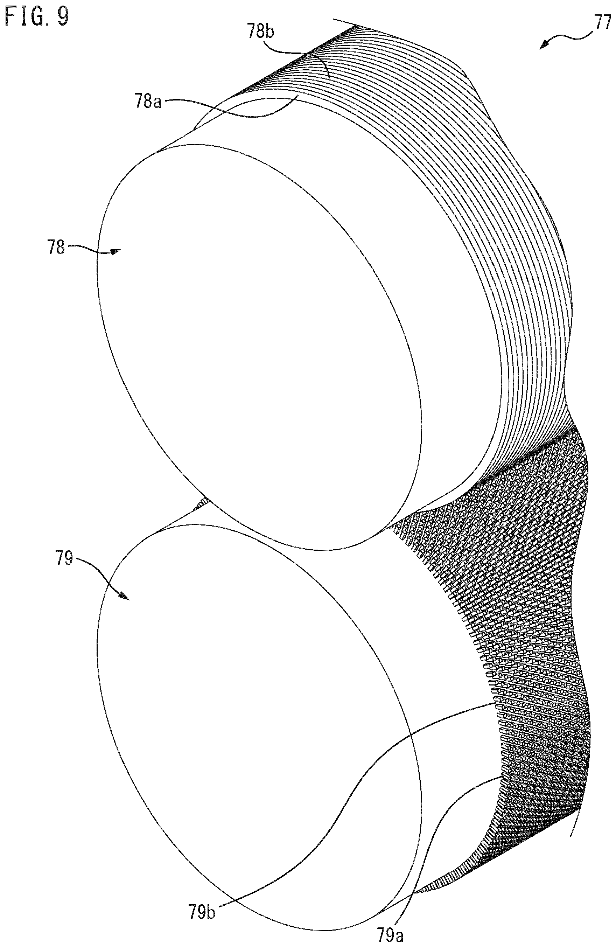

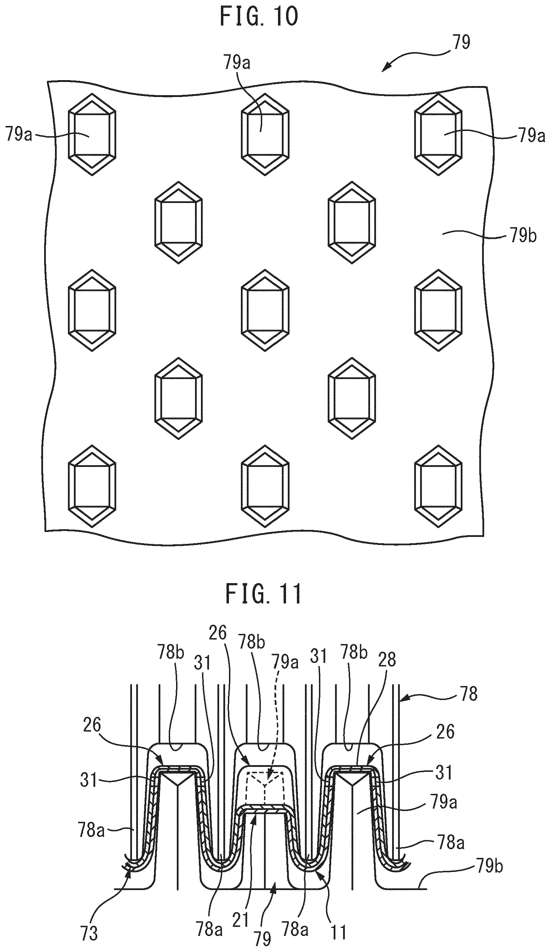

As shown in Fig. 9, the upper stretching roll 78 comprises, on the outer peripheral surface at fixed intervals in the widthwise direction of the roll, the aforementioned ridges 78a formed in mutually parallel rows along the outer peripheral surface of the upper stretching roll 78, and the aforementioned rows of grooves 78b formed between adjacent ridges 78a, 78a. Also, the lower stretching roll 79 comprises, on its outer peripheral surface, a plurality of pins 79a provided so as to interlock with the grooves 78b of the upper stretching roll 78, and the outer peripheral surface sections 79b that interlock with its ridges 78a. As shown in Fig. 9, the pins 79a are disposed at a constant interval in the widthwise direction of the roll so as not to contact the ridges 78a of the upper stretching roll 78 (the interval formed by the sections where the pins 79a are not present across the full width of the lower stretching roll 79, according to this embodiment), while being linearly disposed at approximately constant interval along the outer peripheral surface, with respect to the circumferential direction of the roll. Also, as shown in Fig. 10, the lower stretching roll 79 of this embodiment has a construction in which a plurality of pins 79a are disposed in a zigzag fashion around the outer peripheral surface of the lower stretching roll 79.

When carrying out the shaping step, as shown in Fig. 11, the upper stretching roll 78 pushes the sections where the ridges 78a contact with the nonwoven fabric 73 to be processed, into the outer peripheral surface sections 79b of the lower stretching roll 79, thereby shaping the protrusions 11 of the top sheet 2 (the top sheet web 71 at this stage). Meanwhile, the lower stretching roll 79 has its plurality of pins 79a aligned in rows in the circumferential direction, forcing the nonwoven fabric 73 to be processed contacting at the tip sections of the pins 79a, into the corresponding grooves 78b of the upper stretching roll 78. During this time, the sections of the nonwoven fabric 73 to be processed that have been pulled into the grooves 78b without being in contact with the pins 79a are minimally stretched compared to the other sections, and become the first bottom sections 22 of the first recesses 21 of the recesses 12 of the top sheet 2 (top sheet web 71). Also, the sections that were in contact with the tip sections of the pins 79a are strongly forced into the grooves 78b and shaped, thereby forming second recesses 26 of the top sheet 2 (top sheet web 71) comprising perimeter wall sections 27 and second bottom sections 28.

During formation of the second bottom sections 28 of the second recesses 26, the tip sections of the pins 79a push the contacting sections of the nonwoven fabric 73 to be processed into the grooves 78b while the upper stretching roll 78 and lower stretching roll 79 are meshed with the nonwoven fabric 73 to be processed, and therefore the fiber density is essentially higher than at the other sections. Also, since sections are present where no pins 79a are present across the entire roll width of the lower stretching roll 79, sections are formed in the top sheet web 71 where no second recesses 26 are formed across the entire width (see top sheet 2 in Fig. 6) .

Furthermore, the sections of the nonwoven fabric 73 to be processed according to this embodiment, that were in contact with both edges of the tip sections of the pins 79a of the lower stretching roll 79 in the widthwise direction (widthwise direction of the roll), are also subjected to the tension produced when the ridges 78a of the upper stretching roll 78 push the nonwoven fabric 73 to be processed into the outer peripheral surface sections 79b of the lower stretching roll 79, such that the pins 79a shove aside the thermoplastic resin fibers forming the first perimeter wall sections 29 among the perimeter wall sections of the second recesses, or they break the fibers, forming the aforementioned broken fibers with broken ends. This results in formation of holes 31 having perimeter sections that include broken ends of broken fibers, in the second recesses 26. Incidentally, some of the thermoplastic resin fibers remain in a state passing through the interior spaces of the holes 31, with some of the broken ends of the broken fibers extending into the interior spaces of the holes 31. Since the holes 31 are formed in the direction of the nonwoven fabric 73 to be processed that is along the machine direction MD, i.e. the rotational direction of the stretching rolls 78, 79, which is the direction in which the protrusions 11 and the recesses 12 are laid out, the holes 31 are also formed on the first perimeter wall sections 29 which are the peripheral surfaces, along the direction in which the protrusions 11 and the recesses 12 are laid out.

When the shaping step is complete, this completes the top sheet web 71 as shown in Fig. 3, but the top sheet web 71 is then conveyed toward the absorbent body 4 on the carrier sheet 65, for lamination onto the absorbent body 4, and is laminated and bonded on the upper side surface of the absorbent body 4 via an adhesive layer. During this time, in the top sheet web 71, at least some of the second surfaces 2b of the first bottom sections 22 of the first recesses 21, and the second surfaces 2b of the second bottom sections 28 of the second recesses 26, are bonded with the upper side surface of the absorbent body 4, while the second surfaces 2b of the protrusions 11 are not bonded. Each of the steps described above are then carried out to complete the disposable diaper 1 as a product.

The disposable diaper 1 of this embodiment has portions of the first bottom sections 22 joined to portions of the top sheet side surface of the absorbent body 4, but according to the invention, the first bottom sections do not necessarily need to be joined to the absorbent body so long as the top sheet and the absorbent body are stably joined. In this case as well, the second bottom sections must be joined to the absorbent body.

Moreover, according to this embodiment, the distances between the sections of the second surfaces 2b at the top sections 13 of the protrusions 11 and the sections on the top sheet side surface of the absorbent body 4 are smaller than the distances between the sections of the first surfaces 2a of the first bottom sections 22 nearest the absorbent body 4 and the sections on the top sheet side surface of the absorbent body 4, but according to the invention, the relationship between the distances wherein the sections of the second surfaces at the top sections of the protrusions and the sections on the top sheet side surface of the absorbent body and the distances between the sections of the first surfaces of the first bottom sections nearest the absorbent body and the sections on the top sheet side surface of the absorbent body, does not necessarily need to be such a relationship, so long as detachment between the top sheet and the absorbent body can be stably inhibited.

For the embodiment described above, the top sheet 2 has a construction with at least two protrusions 11 situated between each second recess 26 and the other second recesses 26 that are adjacent to that second recess 26 in the widthwise direction, but according to the invention, there is no limitation to such a construction, and the number of protrusions between adjacent second recesses in the widthwise direction may be set as desired. Furthermore, in the top sheet 2 according to the embodiment described above, the construction in which first recesses 21 of the recesses 12 and the first recesses 21 of other recesses adjacent to those recesses 12 are mutually adjacent has a portion that is continuous over the entire width in the widthwise direction, but according to the invention, the top sheet does not necessarily need to have such a construction.

In addition, for this embodiment, the top sheet 2 comprises holes 31 running through the second surfaces 2b, in the pair of first perimeter wall sections 29, 29 of the second recesses 26, but according to the invention, it is not necessary to form holes in the perimeter wall sections so long as it is possible to ensure the flexibility of the protrusions. In addition, for this embodiment, the second surfaces 2b of the second bottom sections 28 are formed flat, but according to the invention, the second surfaces do not necessarily have to be flat so long as it is possible to adequately ensure the bonding strength or liquid permeability in the second bottom sections.

An absorbent article according to another embodiment of the invention will now be described.

According to another embodiment of the invention, the nonwoven fabric used as the top sheet of the absorbent article is a fiber laminate composed of three fiber layers, the fiber laminate (nonwoven fabric) being composed of a second fiber layer forming the skin-facing surface (first surface) of the nonwoven fabric (skin-facing side fiber layer), a third fiber layer including thermoplastic resin fibers and including no cotton, which is adjacent to the non-skin-facing side surface of the second fiber layer (intermediate layer), and a first fiber layer including cotton, which is adjacent to the non-skin-facing side surface of the third fiber layer (non-skin-facing side fiber layer). If the nonwoven fabric has such a construction, then the third fiber layer will be interposed as an intermediate layer between the second fiber layer forming the skin-facing surface of the top sheet and the first fiber layer that includes cotton, thereby separating the second fiber layer and the first fiber layer, and excreted fluid such as urine that has been absorbed and held in the cotton of the first fiber layer will tend to not be transferred to the second fiber layer and therefore to the skin of the wearer, so that the wearer will not perceive a condition of dampness and a feeling of discomfort will be less likely to be produced.

The following construction may be mentioned as a specific example of a three-layer fiber laminate according to another embodiment.

[Skin-facing side fiber layer] (Second fiber layer)

PET/PE core-sheath composite fiber with fineness of 2.8 dtex, fiber length of 45 mm and basis weight of 10 g/m$^2$

[Intermediate layer] (Third fiber layer)

PET/PE core-sheath composite fiber with fineness of 2.8 dtex, fiber length of 45 mm and basis weight of 10 g/m$^2$

[Non-skin-facing side fiber layer] (First fiber layer)

Blend of PET/PE core-sheath composite fiber with fineness of 2.2 dtex, fiber length of 44 mm and basis weight of 8 g/m$^2$, PET/PE core-sheath composite fiber with fineness of 1.7 dtex, fiber length of 45 mm and basis weight of 1 g/m$^2$, and cotton with basis weight of 1 g/m$^2$.

According to yet another embodiment of the invention, the nonwoven fabric used as the top sheet of the absorbent article is a fiber laminate composed of three fiber layers, the fiber laminate (nonwoven fabric) being composed of a second fiber layer forming the skin-facing surface (first surface) of the nonwoven fabric (skin-facing side fiber layer), a first fiber layer including cotton, which is adjacent to the non-skin-facing side surface of the second fiber layer (intermediate layer), and a third fiber layer including thermoplastic resin fibers and including no cotton, which is adjacent to the non-skin-facing side surface of the first fiber layer (non-skin-facing side fiber layer). If the nonwoven fabric has such a construction, the structure will be such that the third fiber layer is interposed between the first fiber layer that contains cotton and the empty spaces of the protrusions of the nonwoven fabric on the second surface side (non-skin-facing side), and therefore it is possible to reliably ensure larger spaces that confine moisture discharged from the absorbent body disposed on the non-skin-facing side of the third fiber layer (where the third fiber layer functions for auxiliary spaces to confine moisture discharged from the absorbent body). As a result, moisture that has been discharged from the absorbent body can be effectively confined in a region more toward the non-skin-facing side of the first fiber layer, and the wearer can be even further protected from feeling mustiness and the like.

The following construction may be mentioned as a specific example of a three-layer fiber laminate according to another embodiment.

[Skin-facing side fiber layer] (Second fiber layer)

PET/PE core-sheath composite fiber with fineness of 2.8 dtex, fiber length of 45 mm and basis weight of 10 g/m$^2$

[Intermediate layer] (First fiber layer)

Blend of PET/PE core-sheath composite fiber with fineness of 2.2 dtex, fiber length of 44 mm and basis weight of 8 g/m$^2$, PET/PE core-sheath composite fiber with fineness of 1.7 dtex, fiber length of 45 mm and basis weight of 1 g/m$^2$, and cotton with basis weight of 1 g/m$^2$.

[Non-skin-facing side fiber layer] (Third fiber layer)

PET/PE core-sheath composite fiber with fineness of 2.8 dtex, fiber length of 45 mm and basis weight of 10 g/m$^2$

In each of the embodiments described above, the thermoplastic resin fibers in the third fiber layer are not particularly restricted, and may be similar to the thermoplastic resin fibers in the first fiber layer or the second fiber layer. Also, according to these embodiments, preferably each fiber layer has the same basis weight for the thermoplastic resin fibers and the thermoplastic resin fibers contained in the second fiber layer and the third fiber layer have greater fiber sizes (finenesses) than the thermoplastic resin fibers in the first fiber layer. If the nonwoven fabric (fiber laminate) has such a construction, the total amount of thermoplastic resin fibers in the first fiber layer (the number of fibers) will necessarily be greater than in the second fiber layer and the third fiber layer, allowing the cotton-containing first fiber layer to have even greater strength. Furthermore, in such a nonwoven fabric, the thermoplastic resin fibers of the first fiber layer will tend to become entangled with the thermoplastic resin fibers of the second fiber layer or the third fiber layer, such that interlayer separation between each of the fiber layers will tend to be prevented even when the fiber layers composing the nonwoven fabric include cotton with relatively high rigidity.

According to yet another embodiment of the invention, the absorbent article has a back surface film on the non-skin-facing side of the back sheet (that is, the side opposite the absorbent body), the back surface film having lower air permeability than the back sheet. If a back surface film with lower air permeability than the back sheet is present on the non-skin-facing side of the back sheet, then an air permeability gradient will be formed in which the air permeability is lower from the back sheet toward the back surface film, such that moisture discharged from the absorbent body toward the non-skin-facing side (that is, the back sheet side) will tend to be drawn into the back surface film through the back sheet, and be eliminated to the exterior of the absorbent article. With an absorbent article according to such an embodiment, it is possible to reduce moisture discharged from the absorbent body to the skin-facing side (i.e. the side opposite the back sheet side), thereby reducing the likelihood of a feeling of mustiness or the like for the wearer.

In an absorbent article according to yet another embodiment of the invention, the absorbent body has compressed sections on the non-skin-facing side surface of the absorbent body. If compressed sections are present on the non-skin-facing side surface of the absorbent body, excreted fluid absorbed and held in the absorbent body will easily pool in the compressed sections on the non-skin-facing side surface of the absorbent body, with little thereof remaining on the skin-facing side surface of the absorbent body, so that it will be possible to reduce moisture discharged from the absorbent body to the skin-facing side, and to make it even less likely that the wearer will feel mustiness or the like.

According to yet another embodiment of the invention, the absorbent article has a lengthwise direction, a widthwise direction and a thickness direction that are mutually perpendicular, the absorbent body having linear compressed sections running in the lengthwise direction and/or the widthwise direction on the non-skin-facing side surface of the absorbent body, and the absorbent body having a plurality of mutually spaced punctiform compressed sections on the skin-facing side surface. If the absorbent body has such linear compressed sections, excreted fluid that has been absorbed and held in the absorbent body will easily pool on the non-skin-facing side surface of the absorbent body, in a state diffused along the linear compressed sections running in the lengthwise direction and/or the widthwise direction, with little thereof remaining on the skin-facing side surface of the absorbent body, and it will be possible to reduce moisture discharged from the absorbent body to the skin-facing side and to make it even less likely that the wearer will feel mustiness. Furthermore, if the absorbent body has such a plurality of punctiform compressed sections, the punctiform compressed sections that have high fiber density will function as liquid-absorbing sections so that excreted fluid such as urine will tend to be absorbed into the absorbent body in a spot-like manner, while being less prone to being discharged to the skin-facing side through the punctiform compressed sections. This will make it possible to reduce moisture discharged from the absorbent body to the skin-facing side, and to further reduce the likelihood of a feeling of mustiness or the like for the wearer.

According to yet another embodiment of the invention, the absorbent article has a lengthwise direction, a widthwise direction and a thickness direction that are mutually perpendicular, the top sheet having a center region running in the lengthwise direction and including the lengthwise axial line of the absorbent article, in a plane view, and a pair of outside regions located at both edges in the lengthwise direction of the center region and running in the lengthwise direction, the absorbent article having joining sections at the sections of the second surface of the recesses of the nonwoven fabric, that join with the absorbent body, the joining sections extending in the lengthwise direction between the nonwoven fabric and the absorbent body and being disposed in a plurality of rows in the widthwise direction, and the joining sections being disposed so that, among the intervals between adjacent joining sections in the widthwise direction, the intervals between joining sections lying in the outside regions of the top sheet are larger than the intervals between joining sections lying in the center region in the widthwise direction of the top sheet. With this type of absorbent article, the pitch of the joining sections lying in the center region of the top sheet to which excreted fluid such as urine has been supplied are disposed more densely than in the outside regions, and it is therefore possible to firmly join the top sheet and the absorbent body at the center region that greatly contributes to absorption of excreted fluids, while also allowing the empty spaces of the protrusions at the center region to be formed denser and in greater number than in the outside regions, thereby helping to reliably ensure empty spaces to hold moisture discharged from the absorbent body.

For this embodiment there was described a tape-type disposable diaper as the absorbent article, but the absorbent article of the invention is not particularly restricted, and for example, it may be a pants-type disposable diaper or sanitary napkin, or an incontinence pad (panty liner), or the like. When the invention is to be applied in an absorbent article, such as a sanitary napkin, it may be subjected to embossing whereby the top sheet and the absorbent body are integrated. Embossing creates firmer joining between the top sheet and the absorbent body, while also increasing the fiber density between the top sheet and the absorbent body, to help improve take up of excreted fluids from the top sheet to the absorbent body.

Examples

The invention will now be explained in greater detail using examples and comparative examples, with the understanding that the invention is not limited only to these examples.

Example 1

(Production of nonwoven fabric)

To a first stage carding apparatus there were supplied PET/PE core-sheath composite fibers (fineness: 2.8 dtex, fiber length: 45 mm) as thermoplastic resin fibers for formation of the second fiber layer, and the PET/PE core-sheath composite fibers were opened in the first stage carding apparatus, to form a first web with a basis weight of 20 g/m$^2$, corresponding to the second fiber layer, on the conveying surface of the conveying apparatus. The formed first web was conveyed downstream in the machine direction, while two types of PET/PE core-sheath composite fibers with different finenesses (composite fibers A with a fineness of 2.2 dtex and a fiber length of 45 mm, and composite fibers B with a fineness of 1.7 dtex and a fiber length of 45 mm) and cotton, for formation of the first fiber layer, were supplied to the second stage carding apparatus, the fibers were opened inside the second stage carding apparatus, and a web with a basis weight of 13 g/m$^2$ corresponding to the first fiber layer (the basis weights of composite fibers A and B being 10 g/m$^2$ and the basis weight of the cotton being 3 g/m$^2$) was formed on the first web being conveyed. The layered web having the second web layered on the first web was conveyed to an air-through system heating apparatus, and the fibers within each web and between the webs were tangled in the heating apparatus, thereby yielding a two-layer fiber laminate having the first fiber layer and the second fiber layer layered.

(Shaping of a structure composed of protrusions and recesses)

The obtained two-layer fiber laminate (nonwoven fabric) was stretched using a shaping apparatus comprising a pair of upper and lower stretching rolls, as shown in Fig. 9 to Fig. 11, to shape a prescribed structure composed of protrusions and recesses in the nonwoven fabric, as shown in Fig. 3 to Fig. 7.

(Production of absorbent article)

An absorbent material comprising pulp with a basis weight of 220 g/m$^2$ and a super-absorbent polymer (SAP) with a basis weight of 156 g/m$^2$ was covered with tissue having a basis weight of 10 g/m$^2$, to obtain an absorbent body. After joining the nonwoven fabric with the shaped structure composed of protrusions and recesses to one side of the obtained absorbent body, as a top sheet, the other side was joined to a moisture-permeable film with a basis weight of 15 g/m$^2$ as a back sheet, to obtain a laminate. The joining was accomplished using a hot-melt adhesive (coating amount: 3 g/m$^2$).

The obtained laminate was then cut to the prescribed shape of an absorbent article to fabricate an absorbent article for Example 1.

Comparative Example 1

An absorbent article was fabricated for Comparative Example 1 in the same manner as Example 1, except that the two-layer fiber laminate (nonwoven fabric) was not subjected to shaping of the structure composed of protrusions and recesses.

Comparative Example 2

An absorbent article was fabricated for Comparative Example 2 in the same manner as Example 1, except that the fiber material for formation of the first fiber layer was a blend (basis weight: 10 g/m$^2$) of two types of PET/PE core-sheath composite fibers with different finenesses (composite fibers A with a fineness of 2.2 dtex and a fiber length of 45 mm, and composite fibers B with a fineness of 1.7 dtex and a fiber length of 45 mm) (i.e., a fiber material containing no cotton was used).

Comparative Example 3

An absorbent article was fabricated for Comparative Example 3 in the same manner as Example 1, except that the fiber material for formation of the first fiber layer was a blend (basis weight: 10 g/m$^2$) of two types of PET/PE core-sheath composite fibers with different finenesses (composite fibers A with a fineness of 2.2 dtex and a fiber length of 45 mm, and composite fibers B with a fineness of 1.7 dtex and a fiber length of 45 mm) (i.e., a fiber material containing no cotton was used), and the two-layer fiber laminate (nonwoven fabric) was not subjected to shaping of the structure composed of protrusions and recesses.

For evaluation of the tendency to produce a condition of dampness or mustiness, the absorbent articles of Example 1 and Comparative Examples 1 to 3 were subjected to the following measurements of transpiration rate (%) at prescribed times after absorption of artificial urine by the absorbent articles. The measurement results for each construction and transpiration rate of the absorbent articles of Example 1 and Comparative Examples 1 to 3 are shown in Table 1.

[Transpiration rate measurement method]

(1) In order to eliminate the effect of the surrounding environment, the absorbent article sample to be measured is set in a thermo-hygrostat at a temperature of 20°C and a humidity of 60%, and allowed to stand in that environment for 5 days (120 hours).

(2) The absorbent article sample is removed from the thermo-hygrostat, and the initial weight $A_0$ (g) of the absorbent article sample is measured.

(3) The absorbent article sample is spread out on a test stage having a horizontal plane, with the top sheet on the upper side, and a cylinder with an inner diameter of 60 mm is placed on the top sheet.

(4) In the cylinder placed on the top sheet there is dropped 80 mL of artificial urine for 10 seconds.

The artificial urine is prepared by dissolving 200 g of urea, 80 g of sodium chloride, 8 g of magnesium sulfate, 3 g of calcium chloride and approximately 1 g of dye (Blue #1) in 10 L of ion-exchanged water.

(5) Upon confirming that all of the artificial urine in the cylinder has been absorbed by the absorbent article sample, the cylinder is removed from the top of the top sheet and the sample weight $A_1$ (g) of the absorbent article immediately after artificial urine absorption is measured.

(6) The absorbent article sample immediately after artificial urine absorption is allowed to stand in a constant atmosphere, and the absorbent article sample weights $W_1$, $W_3$, $W_5$, $W_8$ and $W_{21}$ (g) are each measured after 1 hour, after 3 hours, after 5 hours, after 8 hours and after 21 hours, from the point where the cylinder was removed from the top of the top sheet in (5) above.

(7) The transpiration rates $E_1$, $E_3$, $E_5$, $E_8$ and $E_{21}$ (%) at each elapsed time point for the absorbent article sample is calculated by the following formula (1). [Mathematical Formula 1]

$$E_N \ (\%) = (A_1 - W_N) \times 100 \diagup (A_1 - A_0) \qquad\qquad (1)$$

(N = 1, 3, 5, 8 or 21.)

[Table 1]

Table 1

| | | Example 1 | Comparative Example 1 | Comparative Example 2 | Comparative Example 3 |
|---|---|---|---|---|---|
| Top sheet composition | Skin-facing side fiber layer (second fiber layer) | PET/PE composite fiber | PET/PE composite fiber | PET/PE composite fiber | PET/PE composite fiber |
| | Basis weight (g/m$^2$) | 20 | 20 | 20 | 20 |
| | Non-skin-facing side fiber layer (first fiber layer) | Two types of PET/PE composite fiber + cotton | Two types of PET/PE composite fiber + cotton | Two types of PET/PE composite fiber | Two types of PET/PE composite fiber |
| | Basis weight (g/m$^2$) | 10 + 3 | 10 + 3 | 10 | 10 |
| | Overall basis weight (g/m$^2$) | 33 | 33 | 30 | 30 |
| | Structure composed of protrusions and recesses | Yes | No | Yes | No |
| Transpiration rate (%) | After 1 hour | 1.7 | 2.3 | 1.9 | 2.4 |
| | After 3 hours | 4.0 | 4.8 | 4.1 | 4.7 |
| | After 5 hours | 7.2 | 8.2 | 7.5 | 7.8 |
| | After 8 hours | 10.4 | 11.7 | 11.8 | 12.0 |
| | After 21 hours | 28.1 | 31.6 | 32.8 | 36.4 |

As shown in Table 1, the absorbent article of Example 1 had a low transpiration rate after artificial urine absorption compared to the absorbent articles of Comparative Examples 1 to 3, and was less prone to a condition of dampness or mustiness. In particular, the absorbent article of Example 1 had a low transpiration rate after 8 hours and after 21 hours from absorption of artificial urine, and tended not to exhibit any condition of dampness or mustiness over a prolonged period. On the other hand, the absorbent article of Comparative Example 1 which had no structure composed of protrusions and recesses, the absorbent article of Comparative Example 2 contained no cotton in the first fiber layer, and the absorbent article of Comparative Example 3 which had no structure composed of protrusions and recesses and contained no cotton in the first fiber layer, all exhibited a high transpiration rate after artificial urine absorption, and tended to exhibit a condition of dampness or mustiness.

Incidentally, the absorbent article of the invention is not restricted to the embodiments and examples described above and can incorporate appropriate combinations and modifications within a range that is not outside of the object and gist of the invention. The ordinal terms "first" and "second" as used throughout the present description serve merely to distinguish between the numbered embodiments and are not used to indicate any relative ordering, precedence or importance.

Reference Sign List

1      Disposable diaper (absorbent article)
2      Top sheet
3      Back sheet
4      Absorbent body
11    Protrusion
12    Recess
13    Top section
14    Empty space
21    First recess

22 First bottom section
26 Second recess
27 Perimeter wall section
28 Second bottom section
29 First perimeter wall section
30 Second perimeter wall section
31 Hole
50 Production apparatus

**Claims**

1. An absorbent article (1) including a liquid-permeable top sheet (2) situated on a side facing a skin of a wearer, a liquid-impermeable back sheet (3) situated on a side not facing the skin of the wearer, and an absorbent body (4) situated between both sheets,

   wherein the top sheet (2) is a nonwoven fabric comprising at least two fiber layers including a first fiber layer (201) composed of cotton and thermoplastic resin fibers and a second fiber layer (202) composed of hydrophobic thermoplastic resin fibers,
   the nonwoven fabric having a first surface (2a) which is a skin-facing side surface, formed by the second fiber layer, and a second surface (2b) which is a non-skin-facing side surface, facing the absorbent body, the nonwoven fabric being provided with a plurality of protrusions (11) protruding toward the first surface (2a) and a plurality of recesses (12) depressed toward the second surface (2b), and formed between adjacent protrusions,
   each of the protrusions (11) having an empty space (14) faced by the second surface of the nonwoven fabric.

2. The absorbent article according to claim 1, wherein the back sheet (3) has air permeability.

3. The absorbent article according to claim 1 or 2, wherein the first fiber layer (201) includes a cotton fiber mass.

4. The absorbent article according to any one of claims 1 to 3, wherein the absorbent article (1) has a back surface film on the non-skin-facing side of the back sheet (3), the back surface film having lower air permeability than the back sheet.

5. The absorbent article according to any one of claims 1 to 4, wherein

   the protrusions (11) are laid out in a first direction on the first surface (2a) of the nonwoven fabric and provided at predetermined intervals in a second direction that is perpendicular to the first direction,
   the recesses (12) are laid out in the first direction and provided between adjacent protrusions (11) in the second direction, the recesses each having a first recess (21) comprising a first bottom section (22) situated more toward the second surface side than the location of the first surface side, at top sections of the protrusions, and a plurality of second recesses (26) inside the first recess (21), provided in a discontinuous manner in the first direction, and depressed from the first bottom section (22) toward the second surface (2b), and
   the second recesses (26) comprise perimeter wall sections (27) extending from the first bottom section (22) in the direction toward the second surface side, and second bottom sections (28) having the highest fiber density of the nonwoven fabric, provided on the edges of the second surface sides of the perimeter wall sections (27), so as to plug the edges.

6. The absorbent article according to claim 5, wherein a pitch of the second recesses (26) in the second direction is no greater than 2.0 mm.

7. The absorbent article according to any one of claims 1 to 6, wherein the protrusions (11) are laid out in the first direction of the first surface (2a) of the nonwoven fabric and are provided at predetermined intervals in a second direction that is perpendicular to the first direction, while the protrusions are laid out up to at least one of the edges in the first direction of the absorbent body, or exceeding at least one of the edges.

8. The absorbent article according to any one of claims 1 to 7, wherein the absorbent body has compressed sections on the non-skin-facing side surface.

9. The absorbent article according to claim 8, wherein

the absorbent article (1) has a lengthwise direction, a widthwise direction and a thickness direction that are mutually perpendicular,
the compressed sections are provided on the non-skin-facing side surface of the absorbent body, as linear compressed sections running in the lengthwise direction and/or the widthwise direction, and
the absorbent body has a plurality of mutually spaced punctiform compressed sections on the skin-facing side surface.

10. The absorbent article according to any one of claims 1 to 9, wherein

the absorbent article (1) has a lengthwise direction (L), a widthwise direction (W) and a thickness direction (T) that are mutually perpendicular,
the top sheet (2) having a center region running in the lengthwise direction and including the lengthwise axial line of the absorbent article, in a plane view, and a pair of outside regions located at both edges in the lengthwise direction of the center region and running in the lengthwise direction,
the absorbent article having joining sections at the sections of the second surface of the recesses (12) of the nonwoven fabric, that join with the absorbent body (4),
the joining sections extending in the lengthwise direction between the nonwoven fabric and the absorbent body and being disposed in a plurality of rows in the widthwise direction, and
the joining sections being disposed so that, among the intervals between adjacent joining sections in the widthwise direction, the intervals between joining sections lying in the outside regions of the top sheet are larger than the intervals between joining sections lying in the center region in the widthwise direction of the top sheet.

**Patentansprüche**

1. Absorbierender Artikel (1), der Folgendes einschließt: eine flüssigkeitsdurchlässige obere Lage (2), die sich auf einer Seite befindet, die einer Haut eines Trägers zugewandt ist, eine flüssigkeitsundurchlässige hintere Lage (3), die sich auf einer Seite befindet, die nicht der Haut des Trägers zugewandt ist, und einen Saugkörper (4), der sich zwischen beiden Lagen befindet,

wobei die obere Lage (2) ein Vliesstoff ist, der mindestens zwei Faserschichten umfasst, die eine erste Faserschicht (201), die aus Baumwolle und thermoplastischen Harzfasern besteht, und eine zweite Faserschicht (202), die aus hydrophoben thermoplastischen Harzfasern besteht, einschließen,
wobei der Vliesstoff Folgendes aufweist: eine erste Oberfläche (2a), die eine Oberfläche der der Haut zugewandten Seite ist, die durch die zweite Faserschicht gebildet ist, und eine zweite Oberfläche (2b), die eine Oberfläche der nicht der Haut zugewandten Seite ist, die dem Saugkörper zugewandt ist, wobei der Vliesstoff mit einer Vielzahl von Erhebungen (11), die sich in Richtung der ersten Oberfläche (2a) erheben, und einer Vielzahl von Vertiefungen (12), die in Richtung der zweiten Oberfläche (2b) abgesenkt und zwischen benachbarten Erhebungen ausgebildet sind, bereitgestellt ist,
wobei jede der Erhebungen (11) einen leeren Raum (14) aufweist, dem die zweite Oberfläche des Vliesstoffs zugewandt ist.

2. Absorbierender Artikel nach Anspruch 1, wobei die hintere Lage (3) Luftdurchlässigkeit aufweist.

3. Absorbierender Artikel nach Anspruch 1 oder 2, wobei die erste Faserschicht (201) eine Baumwollfasermasse einschließt.

4. Absorbierender Artikel nach einem der Ansprüche 1 bis 3, wobei der absorbierende Artikel (1) einen hinteren Oberflächenfilm auf der nicht der Haut zugewandten Seite der hinteren Lage (3) aufweist, wobei der hintere Oberflächenfilm eine geringere Luftdurchlässigkeit aufweist als die hintere Lage.

5. Absorbierender Artikel nach einem der Ansprüche 1 bis 4, wobei

die Erhebungen (11) in einer ersten Richtung auf der ersten Oberfläche (2a) des Vliesstoffs ausgelegt und in vorgegebenen Abständen in einer zweiten Richtung, die senkrecht zu der ersten Richtung ist, bereitgestellt sind,
die Vertiefungen (12) in der ersten Richtung ausgelegt und zwischen benachbarten Erhebungen (11) in der zweiten Richtung bereitgestellt sind, wobei die Vertiefungen jeweils Folgendes aufweisen: eine erste Vertiefung (21), die einen ersten unteren Abschnitt (22), der sich weiter in Richtung der zweiten Oberflächenseite befindet als der Stelle der ersten Oberflächenseite, an oberen Abschnitten der Erhebungen, umfasst, und eine Vielzahl von zweiten Vertiefungen (26) innerhalb der ersten Vertiefung (21), die in einer diskontinuierlichen Weise in der ersten Richtung bereitgestellt und von dem ersten unteren Abschnitt (22) in Richtung der zweiten Oberfläche (2b) versenkt sind,

die zweiten Vertiefungen (26) Folgendes umfassen: Umfangswandabschnitte (27), die sich von dem ersten unteren Abschnitt (22) in der Richtung zu der zweiten Oberflächenseite erstrecken, und zweite untere Abschnitte (28), die die höchste Faserdichte des Vliesstoffs aufweisen, die an den Rändern der zweiten Oberflächenseiten der Umfangswandabschnitte (27) bereitgestellt sind, sodass sie die Ränder ausfüllen.

6. Absorbierender Artikel nach Anspruch 5, wobei ein Abstand der zweiten Vertiefungen (26) in der zweiten Richtung nicht größer als 2,0 mm ist.

7. Absorbierender Artikel nach einem der Ansprüche 1 bis 6, wobei die Erhebungen (11) in der ersten Richtung der ersten Oberfläche (2a) des Vliesstoffs ausgelegt sind und in vorgegebenen Abständen in einer zweiten Richtung, die senkrecht zu der ersten Richtung ist, bereitgestellt sind, während die Erhebungen bis zu mindestens einem der Ränder in der ersten Richtung des Saugkörpers ausgelegt sind oder mindestens einen der Ränder überschreiten.

8. Absorbierender Artikel nach einem der Ansprüche 1 bis 7, wobei der Saugkörper komprimierte Abschnitte auf der Oberfläche der nicht der Haut zugewandten Seite aufweist.

9. Absorbierender Artikel nach Anspruch 8, wobei

der absorbierende Artikel (1) eine Längsrichtung, eine Breitenrichtung und eine Dickenrichtung aufweist, die zueinander senkrecht liegen,
die komprimierten Abschnitte auf der Oberfläche der nicht der Haut zugewandten Seite des Saugkörpers als lineare komprimierte Abschnitte, die in der Längsrichtung und/oder der Breitenrichtung verlaufen, bereitgestellt sind und
der Saugkörper eine Vielzahl von zueinander beabstandeten punktförmigen komprimierten Abschnitten auf der Oberfläche der der Haut zugewandten Seite aufweist.

10. Absorbierender Artikel nach einem der Ansprüche 1 bis 9, wobei

der absorbierende Artikel (1) eine Längsrichtung (L), eine Breitenrichtung (W) und eine Dickenrichtung (T) aufweist, die zueinander senkrecht liegen,
die obere Lage (2) Folgendes aufweist: einen mittleren Bereich, der in der Längsrichtung verläuft und die axiale Längslinie des absorbierenden Artikels, in einer Draufsicht, einschließt, und ein Paar von äußeren Bereichen, die sich an beiden Rändern in der Längsrichtung des mittleren Bereichs befinden und in der Längsrichtung verlaufen,
der absorbierende Artikel Verbindungsabschnitte an den Abschnitten der zweiten Oberfläche der Vertiefungen (12) des Vliesstoffs aufweist, die sich mit dem Saugkörper (4) verbinden,
die Verbindungsabschnitte sich in der Längsrichtung zwischen dem Vliesstoff und dem Saugkörper erstrecken und in einer Vielzahl von Reihen in der Breitenrichtung angeordnet sind und
die Verbindungsabschnitte derart angeordnet sind, dass unter den Abständen zwischen benachbarten Verbindungsabschnitten in der Breitenrichtung die Abstände zwischen Verbindungsabschnitten, die in den äußeren Bereichen der oberen Lage liegen, größer sind als die Abstände zwischen Verbindungsabschnitten, die in dem mittleren Bereich in der Breitenrichtung der oberen Lage liegen.

## Revendications

1. Article absorbant (1) comprenant une feuille de dessus perméable aux liquides (2) se trouvant sur un côté orienté vers la peau d'un utilisateur, une feuille de support imperméable aux liquides (3) se trouvant sur un côté non orienté vers une peau de l'utilisateur, et un corps absorbant (4) se trouvant entre les deux feuilles,

dans lequel la feuille de dessus (2) est un tissu non tissé comportant au moins deux couches de fibres comprenant une première couche de fibres (201) composée à partir de fibres de résine thermoplastique et de coton et une deuxième couche de fibres (202) composée à partir de fibres de résine thermoplastique hydrophobe,
le tissu non tissé ayant une première surface (2a) qui est une surface côté orienté vers la peau, formée par la deuxième couche de fibres, et une deuxième surface (2b) qui est une surface côté non orienté vers la peau, orientée vers le corps absorbant, le tissu non tissé comportant une pluralité de parties saillantes (11) faisant saillie vers la première surface (2a) et une pluralité d'évidements (12) en retrait vers la deuxième surface (2b), et formés entre des parties saillantes adjacentes,
chacune des parties saillantes (11) ayant un espace vide (14) vers lequel est orientée la deuxième surface du tissu non tissé.

2.  Article absorbant selon la revendication 1, dans lequel la feuille de support (3) présente une perméabilité à l'air.

3.  Article absorbant selon la revendication 1 ou la revendication 2, dans lequel la première couche de fibres (201) comprend une masse de fibres de coton.

4.  Article absorbant selon l'une quelconque des revendications 1 à 3, dans lequel l'article absorbant (1) a un film superficiel de support sur le côté non orienté vers la peau de la feuille de support (3), le film superficiel de support ayant une perméabilité à l'air inférieure par rapport à la feuille de support.

5.  Article absorbant selon l'une quelconque des revendications 1 à 4, dans lequel

    les parties saillantes (11) sont disposées dans une première direction sur la première surface (2a) du tissu non tissé et mises en oeuvre selon des intervalles prédéterminés dans une deuxième direction qui est perpendiculaire par rapport à la première direction,
    les évidements (12) sont disposés dans la première direction et mis en oeuvre entre des parties saillantes adjacentes (11) dans la deuxième direction, les évidements ayant chacun un premier évidement (21) comportant une première section de fond (22) se trouvant plus vers le côté de la deuxième surface par rapport à l'emplacement du côté de la première surface, au niveau de sections supérieures des parties saillantes, et une pluralité de deuxièmes évidements (26) à l'intérieur du premier évidement (21), mis en oeuvre de manière discontinue dans la première direction, et en retrait depuis la première section de fond (22) vers la deuxième surface (2b), et
    les deuxièmes évidements (26) comportent des sections de paroi périmétrique (27) s'étendant depuis la première section de fond (22) dans la direction vers le côté de la deuxième surface, et des deuxièmes sections de fond (28) ayant la densité de fibres la plus élevée du tissu non tissé, mises en oeuvre sur les bords des côtés de la deuxième surface des sections de paroi périmétrique (27), de manière à boucher les bords.

6.  Article absorbant selon la revendication 5, dans lequel un pas des deuxièmes évidements (26) dans la deuxième direction n'est pas supérieur à 2,0 mm.

7.  Article absorbant selon l'une quelconque des revendications 1 à 6, dans lequel les parties saillantes (11) sont disposées dans la première direction de la première surface (2a) du tissu non tissé et sont mises en oeuvre selon des intervalles prédéterminés dans une deuxième direction qui est perpendiculaire par rapport à la première direction, alors que les parties saillantes sont disposées jusqu'à au moins l'un des bords dans la première direction du corps absorbant, ou dépassant au moins l'un des bords.

8.  Article absorbant selon l'une quelconque des revendications 1 à 7, dans lequel le corps absorbant a des sections comprimées sur la surface côté non orienté vers la peau.

9.  Article absorbant selon la revendication 8, dans lequel

    l'article absorbant (1) a une direction allant dans le sens de la longueur, une direction allant dans le sens de la largeur et une direction allant dans le sens de l'épaisseur qui sont mutuellement perpendiculaires,
    les sections comprimées sont mises en oeuvre sur la surface côté non orienté vers la peau du corps absorbant, sous la forme de sections comprimées linéaires allant dans la direction allant dans le sens de la longueur et/ou dans la direction allant dans le sens de l'épaisseur, et
    le corps absorbant a une pluralité de sections comprimées réparties de manière ponctuelle et mutuellement espacées sur la surface côté orienté vers la peau.

10. Article absorbant selon l'une quelconque des revendications 1 à 9, dans lequel

l'article absorbant (1) a une direction allant dans le sens de la longueur (L), une direction allant dans le sens de la largeur (W) et une direction allant dans le sens de l'épaisseur (T) qui sont mutuellement perpendiculaires, la feuille de dessus (2) ayant une région centrale allant dans la direction allant dans le sens de la longueur et comprenant la ligne axiale dans le sens de la longueur de l'article absorbant, dans une vue en plan, et une paire de régions extérieures se trouvant au niveau des deux bords dans la direction allant dans le sens de la longueur de la région centrale et allant dans la direction allant dans le sens de la longueur,

l'article absorbant ayant des sections d'assemblage au niveau des sections de la deuxième surface des évidements (12) du tissu non tissé, à des fins d'assemblage avec le corps absorbant (4),

les sections d'assemblage s'étendant dans la direction allant dans le sens de la longueur entre le tissu non tissé et le corps absorbant et étant disposées dans une pluralité de rangées dans la direction allant dans le sens de la largeur, et

les sections d'assemblage étant disposées de telle sorte que, parmi les intervalles entre des sections d'assemblage adjacentes dans la direction allant dans le sens de la largeur, les intervalles entre des sections d'assemblage reposant dans les régions extérieures de la feuille de dessus sont plus grands par rapport aux intervalles entre des sections d'assemblage reposant dans la région centrale dans la direction allant dans le sens de la largeur de la feuille de dessus.

FIG. 1

FIG. 2

EP 3 254 653 B1

FIG. 3

# FIG. 4

# FIG. 5

FIG. 6

# FIG. 7

EP 3 254 653 B1

FIG. 8

FIG. 9

## FIG. 10

## FIG. 11

**EP 3 254 653 B1**

**REFERENCES CITED IN THE DESCRIPTION**

**Patent documents cited in the description**

- JP 2009148328 A
- EP 2277485 A1
- JP 2006320355 A
- WO 2013005782 A1
- EP 2505173 A1
- JP 2005324010 A